(19)

Europäisches Patentamt
European Patent Office
Office européen des brevets

(11)  **EP 4 299 556 B1**

(12)  **EUROPEAN PATENT SPECIFICATION**

(45) Date of publication and mention
of the grant of the patent:
**17.06.2026  Bulletin 2026/25**

(51) International Patent Classification (IPC):
**C07C 69/82** (2006.01)   **C08J 11/22** (2006.01)
**C08J 11/16** (2006.01)   **C07C 67/31** (2006.01)
**C07C 69/80** (2006.01)   **C07C 67/62** (2006.01)
**C08J 11/24** (2006.01)   **C07C 67/03** (2006.01)

(21) Application number: **22788399.8**

(22) Date of filing: **12.04.2022**

(52) Cooperative Patent Classification (CPC):
(C-Sets available)
**C07C 67/03;** C07C 2601/14; Y02W 30/62   (Cont.)

(86) International application number:
**PCT/KR2022/005282**

(87) International publication number:
**WO 2022/220543 (20.10.2022 Gazette 2022/42)**

(54) **METHOD FOR PRODUCING TEREPHTHALATE DERIVATIVE BY TRANSESTERIFICATION OF DIMETHYL TEREPHTHALATE**

VERFAHREN ZUR HERSTELLUNG EINES TEREPHTHALATDERIVATS DURCH UMESTERUNG VON DIMETHYLTEREPHTHALAT

PROCÉDÉ DE PRODUCTION D'UN DÉRIVÉ DE TÉRÉPHTALATE PAR TRANSESTÉRIFICATION DE TÉRÉPHTALATE DE DIMÉTHYLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.04.2021  KR 20210047271**
**06.01.2022  KR 20220002282**
**06.01.2022  KR 20220002283**

(43) Date of publication of application:
**03.01.2024  Bulletin 2024/01**

(73) Proprietor: **Korea Research Institute of Chemical Technology**
**Daejeon 34114 (KR)**

(72) Inventors:
• **CHO, Joungmo**
**Daejeon 34114 (KR)**
• **KIM, Tae Hun**
**Daejeon 34114 (KR)**
• **PHAM, Dinh Duong**
**Daejeon 34114 (KR)**

(74) Representative: **Isarpatent**
**Patent- und Rechtsanwälte**
**Barth Hassa Peckmann & Partner mbB**
**Friedrichstraße 31**
**80801 München (DE)**

(56) References cited:
JP-A- 2012 131 729    JP-A- 2014 070 132
JP-A- H09 296 036    JP-B2- 4 602 469
JP-B2- 4 880 918

• **PHAM DUONG DINH ET AL: "Low-energy catalytic methanolysis of poly(ethyleneterephthalate)", vol. 23, no. 1, 18 January 2021 (2021-01-18), GB, pages 511 - 525, XP055912533, ISSN: 1463-9262, Retrieved from the Internet <URL:https://pubs.rsc.org/en/content/articlepdf/2021/gc/d0gc03536j> DOI: 10.1039/D0GC03536J**

**(Cont. next page)**

- **PHAM DUONG DINH, CHO JOUNGMO: "Low-energy catalytic methanolysis of poly(ethyleneterephthalate)", GREEN CHEMISTRY, vol. 23, no. 1, 18 January 2021 (2021-01-18), GB , pages 511 - 525, XP055912533, ISSN: 1463-9262, DOI: 10.1039/D0GC03536J**

(52) Cooperative Patent Classification (CPC): (Cont.)

C-Sets
**C07C 67/03, C07C 69/82**

## Description

### Technical Field

The present disclosure relates to a transesterification reaction of producing, from dimethyl terephthalate derivative (DMT), value-added derivatives in which functional groups bonded to terephthalate are substituted with different forms of substituents, and to an efficient method of depolymerizing a polymer containing an ester functional group using the transesterification reaction. More particularly, the present disclosure relates to an efficient high-yield conversion method of highly efficiently producing value-added terephthalate derivatives through a transesterification reaction at temperature in a range of from room temperature (25°C) to the boiling point of an alcohol used by using, as a catalyst, one or more materials selected from alkali metal carbonates, alkali hydroxides, alkali metal alkoxides, alkaline earth metal oxides, and guanidine-based organic compounds in the transesterification reaction using DMT and monohydric and/or polyhydric alcohols as raw materials, and also relates to a depolymerization reaction process of a polymer containing an ester functional group, to produce value-added terephthalate derivatives in a high yield and to improve process efficiency and economic efficiency of the transesterification reaction by associating the transesterification reaction with a methanolysis reaction.

### Background Art

Plastic is an inexpensive and durable material, and has advantages in that it is convenient to mold and process. For these reasons, various products are made from plastics. Thanks to these advantages, plastic consumption in industry and our lives has increased dramatically over the past few decades. However, plastic waste that has not been managed properly is left in the environment, causing environmental pollution, and microplastics resulting from breaking down of plastics drift around the ecosystem and accumulate in organisms, eventually entering the human body through fine dust, drinking water, and food. More than 50% of these plastic products are used for single use. That is, most of the plastics are used in single-use applications such as packaging, agricultural films, disposable consumer goods, etc. or in short-lived products that are discarded within one year of manufacture.

Most of the discarded plastics are inadvertently discharged to designated landfills or natural habitats where natural purification is difficult, thereby intensifying the seriousness of environmental pollution day by day. Even naturally degradable or biodegradable plastics can persist for decades, depending on local environmental factors such as level of UV exposure, temperature, presence of microorganisms, and the like.

Recognizing this problem, various efforts are being made to minimize the accumulation of plastics or to reduce their environmental impact, including development of a new plastic material with a short decomposition cycle in natural environment, development of technology for chemically decomposing existing petroleum-based plastics, and development of technology for physical recycling and reprocessing of plastics.

Polymers containing ester functional groups can be depolymerized into monomers, and various chemical reaction pathways have been developed for depolymerization of ester-containing polymers. The monomers generated through polymer decomposition can theoretically have properties equivalent to those of the raw materials initially used for polymer synthesis. Depolymerization pathways that are industrially applicable to recycling polyester include hydrolysis, glycolysis, methanolysis, and ammonolysis. Various chemical depolymerization methods are widely used, ranging from complex processes that take advantage of the advantages of each pathway.

The depolymerization of an ester-containing polymer will be described in more detail. Hydrolysis is known as the process in which decomposition occurs in the presence of a catalyst such as an acid, a base, or a metal salt. In the case of a reaction in which an acid is used as a catalyst, the following disadvantage is pointed out: a very high concentration of sulfuric acid solution is required to obtain a high reaction yield, resulting in problems in terms of process design, operation, and post-treatment. Reaction systems using base and metal salt as a catalyst are inefficient because the decomposition reaction rate is very slow, product purity is low, and catalyst recovery is difficult.

Glycolysis is a depolymerization reaction in which glycol is added as a reactant. The most common example of glycolysis is the process of producing bis-(2-hydroxyethyl) terephthalate (BHET) by adding an excessive amount of ethylene glycol, which is one of the monomer raw materials. Since ethylene glycol, which is one of the raw materials for synthesis of polyethylene terephthalate (PET), is used as a reactant, products produced by depolymerization have a chemical structure in which ethylene glycol is bound to each end of terephthalate. Therefore, a very advantageous result can be obtained in terms of reaction kinetics even though only some of the raw materials of the existing condensation polymerization process using terephthalic acid are replaced.

In addition, since the product obtained by the glycolysis reaction can be directly used as a raw material as it is for PET synthesis, the glycolysis reaction has the advantage of being applicable to the production of polymer materials by modifying only a part of the existing PET production line without considerable investment for additional equipment. Glycolysis is generally carried out under reflux conditions of glycol, which is a reactant. In addition, the rate of decomposition of oligomers to monomers is slow, and an equilibrium state between compounds is reached even though the reaction time is delayed, resulting in low-purity products. Therefore, it is not easy to separate high-purity monomers as end products from the reactants in high yield. In general, metal salts such as zinc acetate or lithium acetate are used as reaction catalysts. Metal ions constituting these catalysts may not be completely removed during the refining process and may remain in the product. Since even a small number of metals harmful to the human body can be included in the recycled monomers, the monomers are difficult to apply to application products produced by reprocessing the monomers, for example, products used especially for food and medical use, household goods, and other household items. In addition, although the reaction proceeds at a high temperature in glycolysis, the recovery and purification process of the product generally follows a low-temperature recrystallization method. Therefore, energy consumption is high and the cost and efficiency of the heat supply from a heat source are low in the production process.

The methanolysis process is one of the processes that have been widely used in actual commercial production processes, not only in global chemical companies in the past but also to small and medium-sized plastic industries. Theoretically, dimethyl terephthalate (DMT) is obtained as a final monomer product by the above-described process, and as the transesterification reaction proceeds, an amount of ethylene glycol equivalent to the number of moles of decomposed terephthalate may be released. In actual reactions, hydroxyethylmethyl terephthalate (1-(2-hydroxyethyl)4-methyl terephthalate) (HEMT) and monomethyl terephthalate (MMT) are produced by side reactions such as partial methanolysis, reaction equilibrium with free ethylene glycol, and hydrolysis. DMT, which is the target compound of the methanolysis reaction, can be used as an intermediate raw material for a process for producing other value-added monomers or a complex hybrid depolymerization process (for example, methanolysis-glycolysis). In addition, DMT has a relatively low boiling point compared to other monomers, and the selectivity for DMT in a hydrogenation reaction can be easily controlled. In addition, it can be used as a gas-phase reactant for the production of value-added diol monomers (for example, 1,4-cyclohexanedimethanol), can be easily purified by a distillation process or a recrystallization process, and can be used as a PET polymerization raw material requiring high purity and high quality. However, since methanol is used as a reaction solvent, severe reaction conditions such as high temperature and high pressure are required, and thus the initial cost for a reactor that can meet the operating environments and related auxiliary facilities with durability is considerable. In addition, since additional unit processes for reactant recovery and product purification are essential, high investment costs may be required. As the catalyst, a depolymerization catalyst that is generally used in a glycolysis reaction, for example, zinc acetate, magnesium acetate, or cobalt acetate, may be used. Alternatively, a transesterification reaction catalyst containing a heavy metal, such as lead dioxide, may be used. However, the use of these catalyst may be harmful to the human body or environment depending on the amount of metal that may remain in the end product.

For depolymerization of polymers containing ester functional groups, methanolysis has been widely used along with hydrolysis or an alkali decomposition process. However, both reactions are high-temperature energy-consuming processes, and when depolymerization is performed at low temperatures, the reaction time is long, and the quality and quantity of the produced end product may be limited. When methanolysis is selected as the reaction pathway for the production of a monomer, the yield and purity of the monomer product, DMT, are greatly affected by the reactivity of the catalyst and reaction impurities. Therefore, in order to reduce the burden of the purification process on the product, both an effective side reaction inhibition method and high reactivity are required.

Japanese Patent Application Publication No. JP1998-287741 (Patent Document 1) as one conventional art discloses a method of recovering each of dimethyl terephthalate and alkylene glycol with high efficiency by processing waste PET with methanol. According to the patent document, when recovering dimethyl terephthalate (DMT) from polyalkylene terephthalate polymer, DMT is produced by a depolymerization reaction of the polyalkylene terephthalate while continuously introducing methanol into the polyalkylene terephthalate which is at least partially in a molten state. In the process, the depolymerization reaction temperature is 200°C to 300°C, and potassium carbonate or the like is used as a catalyst. The technology disclosed in the patent document has a disadvantage in that since the depolymerization temperature is extremely high, the investment cost for equipment and the energy used in the reaction process are considerable.

Therefore, in depolymerizing a polymer containing an ester functional group by methanolysis, it is perferable to use a method capable of improving the depolymerization reaction rate without using excessive energy and of increasing the reaction selectivity to produce DMT in high yield. When a continuous transesterification reaction process can be performed using the DMT as an intermediate, BHET or other types of terephthalate derivatives can be produced in high yield while reducing energy consumption. That is, an efficient and economical process for production of terephthalate derivatives can be implemented.

An example of the above-described reaction is a transesterification reaction using DMT and ethylene glycol as starting materials and producing BHET, which is a value-added monomer, as an end product. In this case, HEMT in which the methyl end group bound to terephthalate is partially substituted is produced as a reaction intermediate, and a compound in which a carboxylic acid functional group is bonded to terephthalate is produced as a byproduct through hydrolysis. An efficient BHET production process can be configured by: designing a reaction pathway that can promote step-by-step transesterification so that all of 2 molar equivalents of methanol bound to DMT can be exchanged by ethylene glycol, while suppressing these side reaction pathways as much as possible; identifying the optimal operating conditions in which BHET generation can proceed predominantly; and applying such optimal operating conditions to the process.

Non-patent Document "Zn- and Ti-Modified Hydrotalcites for Transesterification of Dimethyl Terephthalate with Ethylene Glycol: Effect of the Metal Oxide and Catalyst Synthesis Method"(Amarsinh L. Jadhav, Radhika S. Malkar, and Ganapati D. Ya, ACS Omega 2020, 5, 2088-2096), reported that in performing a transesterification reaction using DMT and ethylene glycol, a composite metal planar structure prepared through modification after introducing zinc and titanium into hydrotalcites was used as a catalyst, so that the selectivity of BHET, a value-added monomer, was able to be increased up to 96.1%

However, in the case of the the technology disclosed in the document, despite the transesterification reaction being performed at a high temperature of 180°C, the conversion rate of dimethyl terephthalate remains at a level of less than 70%, and a large amount of energy may be consumed to purify BHET included in the obtained reaction mixture. In addition, since the catalyst preparation process is complicated, reproducibility of catalyst preparation may be problematic. In addition, since the metal introduced into the catalyst can be leached, there may be limitations in the efficiency and economic feasibility of the process when applied to commercial processes.

Therefore, in the production of value-added terephthalate derivatives through transesterification of DMT, it is important to secure economic feasibility through the use of inexpensive catalysts, process simplification, the use of eco-friendly materials which are not harmful, and the use of not excessive energy. In addition, it is required to develop a reaction and purification combined process that exhibits a high DMT conversion rate and produces terephthalate derivatives in high yield, and which has improved process efficiency and economic feasibility. The produced derivatives can be used as various upcycling raw materials with high industrial applicability, for example, recycled monomers for recycling waste plastic products, additives such as plasticizers, and polymerization raw materials for the synthesis of materials in which part or all of glycol is transformed to improve physical properties.

Dinh Pham et al. (D. Dinh Pham, J. Cho, Low-energy catalytic methanolysis of poly(ethyleneterephtalate), Green Chemistry, 2021, 23, pp. 511-525, DOI: 10.1039/d0gc03536j) describes the depolymerization of polyethylene terephthalate (PET) by methanolysis to afford DMT. The process makes use of methanol, dichloromethane and a catalyst including potassium carbonate. After separation and isolation, DMT undergoes a transesterification reaction on the presence of ethylene glycol and potassium carbonate to afford hydroxyethylmethyl terephthalate (HEMT).

## Disclosure

### Technical Problem

In order to solve the above problems, the present disclosure provides a transesterification reaction method capable of producing value-added monomers in high yield while using a simplified process, that is, a method for preparing different types of terephthalate derivatives from dimethyl terephthalate (DMT).

In addition, the present disclosure expends and diversly applies the aforementioned method of converting DMT into different forms of terephthalate derivatives, thereby providing a method of preparing a terephthalate derivative in high yield at a temperature within a range of from room temperature (25°C) to the boiling point of an alcohol used by using, as reactants, a reaction product generated from depolymerization of a polymer having an ester functional group and a monohydric and/or polyhydric alcohol. That is, the present disclosure provides an efficient and economical depolymerization method capable of preparing a value-added terephthalate derivative through multiple transesterifications using a polymer raw material containing an ester functional group as a starting material.

### Technical Solution

In order to solve the above problems, the present disclosure provides a method of producing a terephthalate derivative from DMT, the method including: (a) carrying out a transesterification reaction by adding a monohydric and/or polyhydric alcohol to DMT as a reactant for transesterification, while applying a flow of a carrier gas in the presence of one or more transesterification catalysts selected from the group consisting of alkali carbonates, alkali hydroxides, alkali alkoxides, alkaline earth metal oxides, and guanidine-based organic; and (b) separating and obtaining a terephthalate derivative produced through the transesterification reaction.

In one embodiment of the present disclosure, the transesterification reaction of step (a) may be carried out at a temperature within a range of from room temperature to the boiling point of the monohydric and/or polyhydric alcohol, which is the solvent, used as a reactant, and a molar ratio of the transesterification catalyst to the dimethyl terephthalate may be in a range of 0.00005 to 1.0.

In one embodiment of the present disclosure, the alcohol in step (a) may be ethylene glycol.

In addition, the present disclosure provides a method of producing a terephthalate derivative by depolymerization of a polymer containing an ester functional group, the method including: (A) depolymerizing the polymer containing an ester functional group by adding methanol, a polar aprotic solvent, and potassium carbonate ($K_2CO_3$), wherein a depolymerization product is obtained including DMT; (B) carrying out a transesterification reaction of the depolymerization product obtained in step (A) by adding a monohydric and/or polyhydric alcohol to the depolymerization product including DMT as a reactant for transesterification while applying a flow of a carrier gas in the presence of one or more transesterification reaction catalysts selected from the group consisting of alkali carbonates, alkali hydroxides, alkali alkoxides to the depolymerization product; and (C) separating and obtaining a terephthalate derivative produced through the reaction.

In one embodiment of the present disclosure, after step (A), a step of separating some compounds from the products of the depolymerization may be further performed, and the separated compounds may include one or more compounds selected from among the polymer containing an unreacted ester functional group, the insoluble catalyst, the polar aprotic solvent, and by-products of the reaction.

In addition, the polar aprotic solvent in step (A) is an inert solvent that does not participate in the depolymerization reaction of the polymer containing an ester functional group, serves to reduce the solubility of the catalyst in alcohol, and is a compound in which at least one of halogen, oxygen, and nitrogen is bonded to an organic compound that has a skeletal structure having a chain form and/or a ring form. The solvent is at least one selected from the group consisting of toluene, xylene, acetone, methyl ethyl ketone, methyl isobutyl ketone, acetonitrile, propionitrile , aminopropionitrile, methylaminopropionitrile, iminodipropionitrile, butyronitrile, methylbutenenitrile, butanenenitrile, methylethylether, diethylether, ethylphenylether, dimethoxybenzene, trimethoxybenzene, methoxyphenol, tetrahydrofuran, methyltetrahydrofuran, dioxane, chloromethane, dichloromethane, chloroform, tetrachloromethane, chlorobenzene, dichlorobenzene, and trichlorobenzene.

In addition, in one embodiment of the present disclosure, in step (A), the number of moles of the alcohol and the number of moles of the polar aprotic solvent with respect to the number of moles of a repeating unit of the polymer containing an ester functional group may be in a range of from 0.1 to 5,000.

In addition, in one embodiment of the present disclosure, before the transesterification reaction in step (B) is carried out, the monohydric and/or polyhydric alcohol reactants as reactants for transesterification of the depolymerization product in step (A), and/or at least one transesterification catalyst selected from the group consisting of alkali carbonates, alkali hydroxides, alkali alkoxides, alkaline earth metal oxides, and guanidine-based organic compounds may be adjusted such that the number of moles thereof is within a predetermined range with respect to the number of moles of the dimethyl terephthalate contained in the depolymerization product. The methanol recovered in step (C) may be reused as a raw material for the depolymerization in step (A).

Further, in one embodiment of the present disclosure, the number of moles of the transesterification catalyst adjusted in step (B) is 0.00005 to 1.0 times the number of moles of the dimethyl terephthalate contained in the depolymerization product.

## Advantageous Effects

The present disclosure provides a method for obtaining a value-added terephthalate derivative from DMT in high yield through a simple process configuration that requires a small amount of transesterification catalyst and low energy consumption. More specifically, a method of changing the reaction equilibrium that can be reached in a closed system by a monomer raw material, a monohydric and/or polyhydric alcohol added, and methanol released by a transesterification reaction is used. That is, the provided is a method of producing a terephthalate derivative in high yield by selectively discharging only methanol to the outside, which inhibits methanolysis corresponding to the reverse reaction and promotes transesterification corresponding to the forward reaction.

For the catalyst as one of the reactants that can be prepared according to an example of the present disclosure, an effective catalyst for a transesterification reaction will be used. The catalyst is not required to have reaction selectivity for methanolysis or a transesterification reaction of producing derivatives. The catalyst is required to exhibit sufficient reactivity and maintain selectivity, with the use of only a trace amount of 1/1000 to 1/10 of the mass of a general catalyst used in a depolymerization reaction of a polymer having an ester functional group. Thus, the catalyst enables an effective and efficient transesterification reaction. Therefore, the transesterification reaction can be carried out with only the entire or partial catalyst remaining in the reaction product discharged from the preceding methanolysis reaction system. The vapor discharged to the outside is high-purity methanol in an amount equal to the equivalent of DMT, and most of it can be concentrated/recovered. 100% of the recovered methanol can be recycled as a reactant for methanolysis for depolymerization of a polymer containing an ester functional group. Therefore, methanolysis and a transesterification reaction that produces derivatives are combined, it becomes a technology that can theoretically completely recycle methanol.

In addition, the present disclosure can provide a method for producing a high-yield terephthalate derivative using DMT and ethylene glycol obtained from depolymerization of a polymer having an ester functional group as reaction intermediates. For example, a BHET production method can be provided in which DMT and ethylene glycol are produced by a low-temperature methanolysis reaction that can be carried out in the presence of methanol, a polar solvent, and a transesterification catalyst, followed by addition of ethylene glycol to the produced DMT, which is called a transesterification reaction, to produce BHET as an end product. When depolymerization of a polymer containing an ester functional group is performed according to an example of the present disclosure, high-yield production of BHET, which is a value-added monomer, is possible without using the high-temperature reaction conditions (190°C to 280°C) required for conventional glycolysis. For example, high-yield BHET can be selectively produced only by a series of reaction processes maintained at a temperature close to room temperature below the boiling point of the applied diol reactant (for example, ethylene glycol) and even below the boiling point of methanol. This means that low-temperature, low-energy glycolysis depolymerization technology can be implemented.

The method according to the present disclosure produces a less amount of dimers or oligomers than conventional high-temperature glycolysis reactions, and thus can produce a relatively high concentration of BHET while maintaining a reaction temperature that is close to the operating temperature range of a purification process for removing impurities (including dimers and oligomers). The method does not involve energy-consuming preheating or cooling during the transfer of reaction products, and directly send the reaction products to a purification process, thereby minimizing energy loss and facilitating a continuous reaction and a post-treatment process.

The transesterification reaction that can be performed according to the present disclosure can be applied to the production of recycled monomers or terephthalate derivatives that can be applied to the synthesis of various types of materials other than BHET that can be used for repolymerization of PET. For example, the prepared monomer can be used to replace phthalate-based plasticizers that are known to cause hormone disruption in the human body due to their geometric feature different from that of the estrogen hormone. In addition, the prepared monomer can be used to produce raw materials for plastics with improved properties. Although PET has excellent thermal and mechanical properties, it is known that it cannot be injection-molded within a short time because of its low final crystallization temperature and slow crystallization rate. The use of PET is limited due to such properties. Accordingly, market demand for various types of polymer resins having improved physical properties suitable for purposes and uses is steadily increasing. In this regard, there are several methods widely used in the industry to improve physical properties of an end product polymer. For example, a new monomer is added to the diol unit of PET for copolymerization, or some of the diols used during condensation polymerization are modified. As an example, when polymerization is performed by replacing ethylene glycol with butylene glycol in the process of condensation polymerization, polybutylene terephthalate (PBT), which is a polymer suitable for injection molding, can be produced according to the characteristics of a more flexible chain structure. In addition, introducing diols of different types can be applied to the synthesis of various types of polymer materials. That is, in performing the transesterification reaction of DMT according to an example of the present disclosure, various types of terephthalate derivatives can be easily produced by modifying reactants such as monohydric and/or polyhydric alcohols and applying similar reaction conditions. In particular, when the reaction product produced from methanolysis of waste PET is directly used as a raw material, recycled monomers can be economically produced through a very simple and easy reaction process. In addition, by varying the feeding of raw materials, it is possible to configure a wide range of upcycling processes capable of flexibly producing various value-added terephthalate derivatives.

## Description of Drawings

FIG. 1 is a schematic diagram of a method for supplying a carrier gas for discharging methanol generated by a transesterification reaction of DMT, to the outside of a reactor according to one embodiment of the present disclosure;
FIG. 2 is a schematic diagram of an example of a process combination that can be configured to obtain a terephthalate derivative, which is a product, in high yield through depolymerization of a polymer raw material containing an ester functional group according to one embodiment of the present disclosure; and

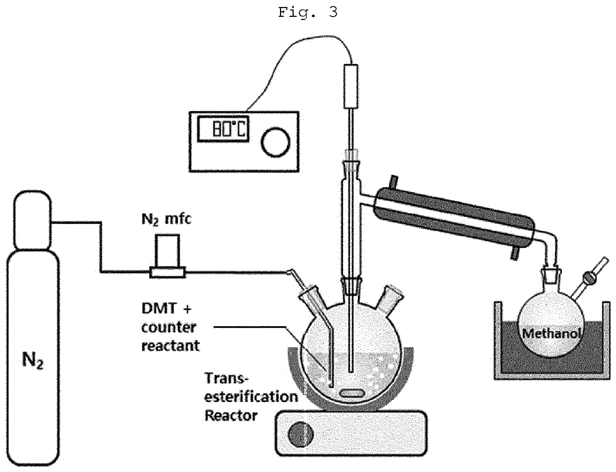

FIG. 3 is a schematic diagram of an example of the configuration of an experimental apparatus for carrying out the transesterification of DMT according to one embodiment of the present disclosure.

## Best Mode

Unless otherwise defined, all terms including technical and scientific terms used herein have the same meaning as commonly understood by those who are ordinarily skilled in the art to which the present disclosure pertains. In general, the nomenclature used herein is one well known and commonly used in the art.

It will be further understood that when a component "comprises" or "has" another component, it means that the component may further include another component, not excluding another component unless stated otherwise.

The present disclosure provides an optimal catalyst for obtaining a value-added terephthalate derivative in high yield by carrying out a transesterification reaction in which DMT is as a raw material and a monohydric and/or polyhydric alcohol is used as an additional reactant.

In one aspect, the present disclosure provides a transesterification reaction method in which one or more materials selected from the group consisting of alkali carbonates, alkali hydroxides, alkali alkoxides, alkaline earth metal oxides, and guanidine-based organic compounds are used as a catalyst for converting DMT into terephthalate derivatives in high yield through transesterification.

In addition, the present disclosure provides a transesterification reaction method capable of producing DMT-originating terephthalate derivatives in high yield. In the method, when converting DMT into terephthalate derivatives through glycolysis, optimal reaction conditions are set as well as optimal transesterification reaction catalysts are selected.

The method of the present disclosure includes: (a) carrying out a transesterification reaction by adding a monohydric and/or polyhydric alcohol to DMT as a reactant for transesterification, while applying a flow of a carrier gas in the presence of one or more transesterification catalysts selected from the group consisting of alkali carbonates, alkali hydroxides, alkali alkoxides, alkaline earth metal oxides, and guanidine-based organic; and (b) separating and obtaining a terephthalate derivative produced through the transesterification reaction.

In the present disclosure, the introduction of the catalyst and the application of the flow of the carrier gas to the reactant in step (a) may be performed without limitation before or after the temperature of the reactant reaches the target temperature, and the reaction can be carried out under a batch reaction condition or a continuous flow condition. The monohydric and/or polyhydric alcohol, which is an additional reactant, and the catalyst may be added as a mixture in which DMT, the alcohol, and the catalyst are mixed, before the preparation for a reaction.

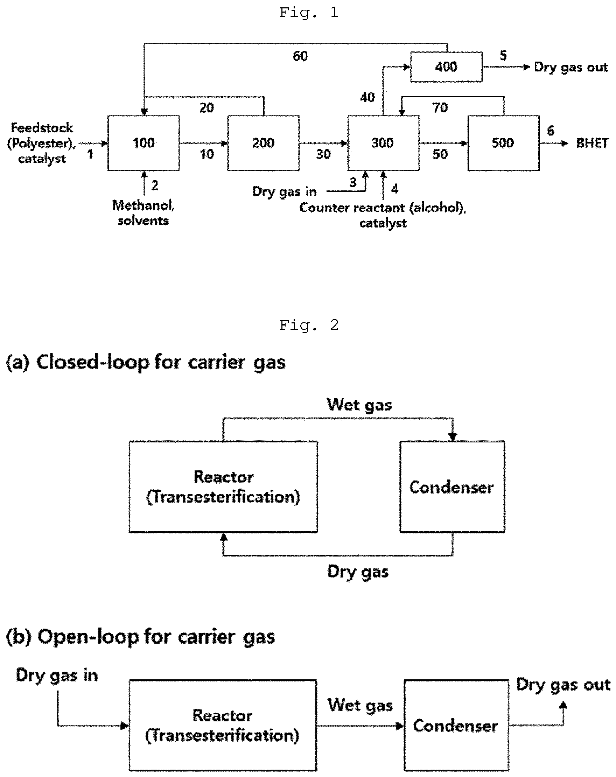

The flow of the carrier gas is used as a means for discharging methanol to the outside, and a flow by forced circulation of a gaseous phase having a pressure gradient may be used. In addition, after condensing the methanol vapor outside the reactor, gas that is near-lean in a vapor phase is recirculated, which is called a continuous recirculation flow (a in FIG. 2). Alternatively, an inert gas may be continuously externally supplied to discharge methanol to the outside (b in FIG. 2).

When carrying out the transesterification reaction in step (a), the dimethyl terephthalate, the monohydric and/or polyhydric alcohol, and the catalyst for transesterification reaction are introduced into the reactor, and a transfer pipe may be placed below the level of the reactants and bubbles are ejected so that the carrier gas remains in direct gas-liquid contact with the reactants in the reactor. In this process, the concentration of methanol vapor in the gas phase is formed, and the methanol can be continuously purged to the outside by the outward flow of the carrier gas. When the methanol vapor is diffused into the carrier gas, it is advantageous that the mass transfer occurs to a concentration equal to higher than the saturation concentration in terms of reactivity. However, if the carrier gas is supplied at a sufficient flow rate, the amount of methanol that is purged may be controlled by the rate of the transesterification reaction.

The temperature of the transesterification reaction is in the range in which the evaporation rate of methanol that is generated by the reaction or is present in the reactant occurs in the gas-liquid contact process, that is, the range of from room temperature to the boiling point of the monohydric and/or polyhydric alcohol used as the reactant. In addition, the pressure may be maintained in a range of 0.1 torr to 5 atm on an absolute pressure basis. However, the temperature and pressure may be changed within the above range considering the range of temperature and pressure for transesterification and the rate of removal of methanol purged by the carrier gas.

The temperature range may vary depending on the transesterification catalyst to be introduced, and is preferably a range of from room temperature to the boiling point of the alcohol used as the reactant, and the pressure is preferably near atmospheric pressure in terms of stable and continuous process operation. In addition, a reaction time in step (a) may vary depending on the amount of DMT and the amount of the alcohol supplied in addition to the type and amount of the catalyst introduced for the reaction.

First, in step (a), the ratio of the number of moles of the transesterification reaction catalyst reaction with respect to the number of moles of the DMT, which is the raw material, is in a range of 0.0001 to 0.2. When the molar ratio of the transesterification reaction catalyst with respect to the DMT, which is the raw material, is less than 0.00005, the transesterification reaction of DMT by the monohydric and/or polyhydric alcohol may proceed slowly, which means low process efficiency. When the molar ratio exceeds 1.0, it is not economical because the degree of improvement compared to the increased in the content of the catalyst is not significant, the amount of by-products may increase, and the excess catalyst serves as an impurity during separation of the terephthalate derivative, thereby affecting the separation and purification efficiency.

The transesterification reaction catalyst is at least one selected from the group consisting of alkali carbonates, alkali hydroxides, alkali alkoxides, alkaline earth metal oxides, and guanidine-based organic compounds. Preferably, the transesterification reaction catalyst may be one or more selected from among $K_2CO_3$, $KHCO_3$, $Na_2CO_3$, $NaHCO_3$, $NaOH$, $KOH$, $MgO$, $CaO$, $CH_3OK$, $CH_3ONa$, and TBD. The amount and combination of catalysts may be determined to significantly improve the conversion rate of DMT and the yield of terephthalate derivatives as well.

In step (a), the amount of ethylene glycol may be in a range of from 1 to 50 moles per mole of DMT. An esterification reaction can occur most efficiently when the amount of ethylene glycol is within the above range.

The monohydric and polyhydric alcohols may be linear, branched, cyclic, or mixed alcohols having 1 to 20 carbon atoms, and the polyhydric alcohols have two or more OH functional groups. For example, one or more alcohols selected from among methanol, ethanol, n-propanol, isopropanol, ethylene glycol, 1,2-propanediol, 1,3-propanediol, 1,2-butanediol, 1,3-butanediol, 1,4-butanediol, glycerol, and 1,2,4-butanetriol may be used. Preferably, ethylene glycol may be used.

In the transesterification reaction of step (a) in which the monohydric and/or polyhydric alcohol is added to DMT in the presence of the transesterification reaction catalyst, a terephthalate derivative and methanol are produced. The reaction is performed under a condition in which methanol vapor is continuously purged to the outside of a reaction system by the carrier gas that is externally supplied to the reactor, so that the forward reaction to the produced terephthalate derivative which is a reaction product can proceed to the extent that the concentration of the terephthalate derivative exceeds the equilibrium concentration. As a method of supplying the carrier gas, as shown in FIG. 2, a one-way flow (b in FIG. 2) in which methanol vapor is separated using a condenser and the carrier gas is then discharged to the outside is used, or a recirculation method (a in FIG. 2) in which methanol vapor is removed and then the carrier gas is recirculated is used.

The carrier gas injected into the reactor is used to make the concentration of methanol in reactants remain low so that the methanol produced as a by-product of the transesterification reaction cannot be recombined or can be less recombined by the reverse reaction. By maintaining such conditions, the DMT conversion rate and the yield of terephthalate derivative as the target product can be improved. In this case, the carrier gas can be used without limitation as long as it does not have a chemical action on the alcohol-added transesterification reaction of DMT or on the catalyst. Preferably, air, nitrogen, argon, helium, etc. may be used. An inert gas that is substantially free of moisture may be advantageously used.

In the present disclosure, step (b) is a step of separating and obtaining the terephthalate derivative formed in the above reaction. Specifically, the terephthalate derivative formed in step (a) is separated to have high purity, from a reaction mixture containing the unreacted residual DMT, the ethylene glycol, the residual catalyst, etc. This step may be carried out using a generally known purification method. Although the method is not limited, a physical method such as filtration, crystallization, centrifugation, evaporation, distillation, etc. and a chemical method such as adsorption, neutralization, and salting out may be used in combination.

In the present disclosure, when producing a value-added terephthalate derivative through an alcohol-added transesterification reaction in which DMT is used as a starting material and monohydric and/or polyhydric alcohols may be used as additional raw materials, the DMT may be obtained from depolymerization of a polymer containing an ester functional group. The terephthalate derivative can be produced by subjecting the depolymerization product which is pretreated or is not pretreated to transesterification with the monohydric and/or polyhydric alcohols.

The monohydric and/or polyhydric alcohol-added transesterification reaction of DMT may be applied to a high-yield terephthalate derivative production method using a two-stage serial reaction composed of methanolysis (primary step) of a polymer containing an ester functional group and the subsequent transesterification (second step) in which a reaction product of the methanolysis is directly used as a raw material.

That is, the present disclosure provides a method of producing a terephthalate derivative by depolymerization of a polymer containing an ester functional group, the method including: (A) depolymerizing the polymer containing an ester functional group by adding an alcohol, a polar aprotic solvent, and potassium carbonate ($K_2CO_3$); (B) carrying out a transesterification reaction while applying a flow of a carrier gas to the depolymerization product obtained in step (A); and (C) separating and obtaining a terephthalate derivative produced through the reaction.

FIG. 1 is a schematic diagram showing a process of preparing a terephthalate derivative from a polymer, as a raw material, containing an ester functional group, through a transesterification reaction. First, in step (A) 100, an alcohol, a polar aprotic solvent, potassium carbonate ($K_2CO_3$), and a polymer containing an ester functional group (hereinafter, simply referred to as ester-containing polymer) are introduced into a depolymerization reactor for depolymerization. The ester-containing polymer comes into contact with the alcohol, the polar aprotic solvent, and the potassium carbonate and is thus converted into an alcohol-added monomer through a transesterification reaction. That is, low temperature depolymerization of the polymer proceeds effectively. The process is very simple, DMT can be obtained in a high yield of 90% or more, and energy consumption is low. Therefore, the process is very economical.

Here, the ester-containing polymer may be used alone or used in combination with waste plastics, such as polyethylene, high-density polyethylene, low-density polyethylene, polypropylene, or a combination thereof. The above-mentioned polymers to be mixed with the ester-containing polymer listed are merely examples, and the polymers to be mixed with the ester-containing polymer are not limited thereto.

In addition, the polymer containing an ester functional group may be a polymer formed by condensation polymerization of a dicarboxylic acid and a dialcohol. The dicarboxylic acid is selected from terephthalic acid, naphthalene dicarboxylic acid, diphenyldicarboxylic acid, diphenyl ether dicarboxylic acid, diphenylsulfonedicarboxylic acid, diphenoxyethanedicarboxylic acid, succinic acid, adipic acid, sebacic acid, Azelaic acid, decanedicarboxylic acid, cyclohexanedicarboxylic acid, trimellitic acid, pyromellitic acid, and combinations thereof. The alcohol is selected from ethylene glycol, trimethylene glycol, 1,2-propanediol, tetramethylene glycol, neopentyl glycol, hexamethylene glycol, decanmethylene glycol, dodecamethylene glycol, 1,4-cyclohexanedimethanol, diethylene glycol, triethylene glycol, tetraethylene glycol, polyethylene glycol, dipropylene glycol, tripropylene glycol, tetrapropylene glycol, polypropylene glycol, di(tetramethylene) glycol, tri(tetramethylene) glycol, polytetramethylene glycol, pentaerythritol, 2,2-bis(4-β-hydroxyethoxyphenyl)propane, and combinations thereof.

For example, the polymer containing an ester functional group may be selected from polyethylene terephthalate (PET), polypropylene terephthalate (PPT), polyglycolide or polyglycolic acid (PGA), polylactic acid (PLA), polycaprolactone (PCL), polyhydroxyalkanoate (PHA), poly hydroxybutyrate (PHB), polyethylene adipate (PEA), polybutylene succinate (PBS), poly(3-hydroxybutyrate-co-3-hydroxyvalerate) (PHBV), polybutylene terephthalate (PBT), polytrimethylene terephthalate (PTT), polyethylene naphthalate (PEN), Vectran, and combinations thereof.

One of the most common examples of the polymer containing an ester functional group is polyethylene terephthalate (PET), and in this case, starting materials for preparing the polymer are ethylene glycol and terephthalic acid or derivative monomers thereof.

The polymer containing an ester functional group used in the present disclosure may be in a state containing various impurities rather than in a pure state. For example, a mixture the polymer containing an ester functional group and debris including, but not limited to, bottle caps, adhesives, paper, residual liquids, dust, or combinations thereof may be used as a depolymerization raw material.

The alcohol as a reactant is methanol.

The number of moles of the alcohol per mole number of the repeating unit of the polymer containing the ester functional group, as a raw material, is in a range of 0.1 to 5,000 and preferably a range of 0.1 to 500.

Potassium carbonate is used as the catalyst. Potassium carbonate, represented by the chemical formula $K_2CO_3$, is a white crystalline material that is soluble in water. It is preferable that as the potassium carbonate, an anhydrate form is used rather than a hydrate form. More preferably, the potassium carbonate is dried before use. The amount of potassium carbonate used may be 0.01 times or more, preferably 0.01 to 100 times, and more preferably 0.1 to 10 times the number of moles of the repeating unit of the polymer containing an ester functional.

The polar aprotic solvent is used as an organic liquid compound that can be applied to lower the activation energy of the ester functional group decomposition reaction or the transesterification reaction, and is a non-reactive, inert solvent that does not directly participate in the reaction. A material that can lower the solubility of the catalyst (potassium carbonate) in the reactant (alcohol) to induce a heterogeneous catalytic reaction and to dissolve the resulting monomer is preferably used in terms of reaction thermodynamics.

The skeletal structure of the organic compound in the polar aprotic solvent may be a chain or cyclic compound, and may have a form in which a halogen element is directly bonded to an organic compound, or organic compounds linked to each other via oxygen or nitrogen. Specifically, for example, at least one selected from toluene, acetone, methyl ethyl ketone, methyl iso-butyl ketone, acetonitrile, tetrahydrofuran, dichloromethane, chloroform, chlorobenzene, methyl phenyl ether, and ethyl phenyl ether may be used.

In addition, the polar aprotic solvent may be used in an amount that falls within a molar ratio range of 0.1 to 5,000 and preferably 1 to 500 with respect to the repeating unit containing an ester group.

Since step (A) depolymerizes the polymer containing an ester functional group at a temperature in a range of 0°C to 80°C and preferably a range of 10°C to 60°C, in some examples of step (A), the polymer can be completely decomposed under conditions of near normal pressure and near room temperature, and a reaction system with a simple structure and a simple configuration can be constructed. Therefore, the investment cost can be much lower than that of existing technologies or existing processes, and efficient and stable energy management is also possible.

On the other hand, since the depolymerization in step (A) uses a basically water-soluble, heterogeneous, low-cost catalyst, it is easy to recover and reuse the catalyst after the reaction, and it is easy to lower the temperature of the reactant to room temperature or lower and to recover the produce monomers at a high recovery rate. In addition, the used solvent can be reintroduced into the process for depolymerization of a fresh raw material polymer. Therefore, the economic efficiency of the recycling process can be further improved.

In addition, in step (A), sufficient energy required for depolymerization can be obtained only by mixing heat, dissolution heat, etc., generated in the process of preparing the mixed solution for the depolymerization reaction without externally inputting heat from an additional heat source. In this case, the depolymerization may be carried out in a heat-insulating reactor. Alternatively, as in some embodiments according to the present disclosure, depolymerization may be performed while supplying heat using an external heat source.

In addition, the depolymerization of the polymer may be performed at atmospheric pressure or higher. Specifically, the depolymerization may be performed at a pressure of about 1 atm to 6.5 atm.

In addition, the depolymerization of the polymer may be performed in the state of being exposed to the air or in a closed system form, or may be performed while refluxing the solvent with the use of a condenser.

In addition, the depolymerization reaction time in step (A) may vary depending on the amount of the polymer used. In the case of room temperature with no energy applied, monomers can be obtained in a sufficiently high yield within 24 hours, and materials other than the reactants do not undergo significant chemical changes so that most of the materials can be recovered and reintroduced into the process.

After the step (A), step 200 of separating some compounds from the depolymerization products obtained by the depolymerization and discharging the compounds to the outside may be further performed. Specifically, the step of separating some of the compounds from the depolymerization products is a step of separating a stream containing at least one selected from the polymer, insoluble catalyst, polar aprotic solvent, reaction by-product, etc., from a reaction mixture 10 of DMT obtained after the completion of the depolymerization reaction of step (A), unreacted substances remaining together with ethylene glycol, methanol, a polar aprotic solvent, a catalyst, or monomethyl terephthalate, and compounds derived from terephthalic acid (TPA), and discharging the stream to the outside of the reactor. This step is performed by a generally known method without particular limitations.

As an example of the separation, when the insoluble catalyst and unreacted polymer are separated from the depolymerization product through filtration, the depolymerization product may take a homogeneous liquid form. When the temperature is increased or the pressure is reduced so that the polar aprotic solvent or methanol can be partially or entirely evaporated or distilled, DMT which is one of the depolymerization products is crystallized. Here, a washing liquid is added to remove foreign substances and to increase the concentration of DMT, and processes such as recrystallization, physical filtration, distillation, evaporation, and drying are additionally performed to obtain a raw material for an ethylene glycol-added transesterification reaction. The solvent and catalyst, etc., separated from the depolymerization product may be reintroduced (20) as raw materials for the methanolysis depolymerization 100, which corresponds to step (A).

Meanwhile, step 200 of separating some of the compounds may be a preparation step for the transesterification step 300 performed by adding monohydric and/or polyhydric alcohol to the produced DMT. Step 200 is not essential but optional. That is, without performing step 200 of separating the depolymerization product after step (A) in which the polymer containing an ester functional group is depolymerized, the depolymerization product can be directly used as a raw material for the subsequent step (B).

In the present disclosure, step (B) 300 is a step of causing a transesterification reaction of the depolymerization product obtained in step (A) while applying a carrier gas stream 3 to the depolymerization product. Since the method of carrying out the transesterification reaction in step (B) is performed by the same method as the method of preparing a terephthalate derivative from DMT, which is described above, a detailed description thereof will be omitted. DMT in the step (B) is a product 30 obtained by depolymerization of a polymer having an ester functional group. The depolymerization can be carried out by a simple process at room temperature and normal pressure. The depolymerization products include DMT produced in high yield and a polyhydric alcohol. This DMT is used in the terephthalate derivative production method of the present disclosure, so that a terephthalate derivative can be obtained as an end product directly from a polymer containing an ester functional group.

Before the transesterification reaction in step (B), a monohydric and/or polyhydric alcohol as a solvent for transesterification; and/or at least one transesterification catalyst selected from the group consisting of alkali carbonates, alkali hydroxides, alkali alkoxides, alkaline earth metal oxides, and guanidine-based organic compounds is added to the depolymerization product of step (A), in an adjusted predetermined molar ratio with respect to the dimethyl terephthalate contained in the depolymerization product. After the adjustment of the molar ratio, the transesterification reaction will be performed.

The control of the number of moles of the alcohol and/or transesterification reaction catalyst is performed by adding the alcohol and/or the transesterification catalyst to the depolymerization product or partially removing the the alcohol and/or the transesterification catalyst. Alternatively, the control may be performed by adding a new monohydric and/or polyhydric alcohol after the partial removal. In this case, the removal process and the addition process may be performed in step 200 of separating some of the compounds described above.

The monohydric and polyhydric alcohols may be linear, branched, cyclic, or mixed alcohols having 1 to 20 carbon atoms, and the polyhydric alcohols have two or more OH functional groups.

The number of moles of ethylene glycol after the adjustment is in a range of 0.1 to 100 per mole of dimethyl terephthalate included in the depolymerization product, and is preferably a range of 1 to 50.

In addition, the number of moles of the transesterification reaction catalyst after the adjustment is in a range of 0.00005 to 1.0 per mole of dimethyl terephthalate included in the depolymerization product, and preferably a range of 0.0001 to **0.2.**

In the transesterification reaction 300, in order to inhibit the reverse reaction caused by methanol and to continue the forward reaction in which the product is produced and the monohydric and/or polyhydric alcohol is added, methanol is discharged from the reactor by the carrier gas stream 3 (40), and the methanol recovered by the condenser 400 maintained at a low temperature can be reused (60) as a raw material for the depolymerization reaction of step (A). Compared to the existing BHET production method using glycolysis performed in a high temperature condition (usually in the range of 200°C to 280°C), the reaction proceeds rapidly even at low temperatures considerably lower than the boiling point of monohydric and/or polyhydric alcohols. Therefore, terephthalate derivative as the end product can be obtained effectively. In addition, products (dimers or oligomers) generated due to insufficient progress in decomposition (or depolymerization) of the product due to different reaction temperature ranges and reaction pathways leading to the products are generated in small amounts or are not generated at all, but the targeted terephthalate derivative can be obtained in high yield.

With the use of the method of the present disclosure, the reactivity in the low temperature region and the efficiency of the purification process are improved, and thus energy consumption can be greatly reduced compared to the existing process. That is, the prevent disclosure provides a means capable of economically producing value-added terephthalate derivatives.

In the present disclosure, step (C) is step 500 of separating and obtaining a high-yield terephthalate derivative generated through the above reaction. This step can be performed in the same way as the method for producing a terephthalate derivative from DMT, which is described above in detail. Therefore, a redundant description will be omitted here.

Hereinafter, details of the process of the present disclosure will be described through comparative examples and examples. The examples and the comparative examples are presented only for illustrative purposes, and the scope of application of the present disclosure is not limited by the following examples.

Raw material 1 (dimethyl terephthalate as raw material)

Dimethyl terephthalate (Sigma-Aldrich, catal.# 185124, purity > 99.0%) supplied from a reagent manufacturer was evenly ground using a mortar and pestle to prepare a finely grained powder as Raw material 1.

Raw material 2 (polymer containing ester functional group, as raw material)

As a polymer containing an ester functional group, a waste polyethylene terephthalate bottle discharged after use was washed to remove any remaining foreign substances, and was then dried and pulverized using a continuous grinding mill (manufacturer and model: IKA MF10.1). Only plastic chips that are 1 to 3 mm long in width and length when measured from the largest surface thereof and which have a thickness of 0.5 mm or less were collected and prepared as Raw material 2.

<Example 1>

About 14.5 g of the DMT prepared as Raw material 1 and about 55.6 g of ethylene glycol (Sigma-Aldrich; purity $\geq$ 99.8%), which is a dihydric alcohol polar solvent, were put into a three-neck flask. Here, 55.6 g of the ethylene glycol corresponds to 12 times the number of moles of Raw material 1. Next, a distillation head jacketed condenser was attached to the flask, and the mixture in the flask agitation was stirred at a speed of 1,200 rpm using a magnetic stirrer.

In order to effectively remove methanol, which is a reaction product, high-purity nitrogen (Central Industrial Gas Co., Ltd.; 99.9992%) was used as an inert carrier gas, and the gas flow rate that comes into contact with the liquid in the reactor was 200 sccm. The value of the gas flow rate that is is normalized to the volume of the initial reaction mixture under standard conditions and converted into a gas hourly space velocity (GHSV ($hr^{-1}$)) was 240. Such a gas flow rate was maintained using a mass flow controller.

Next, when the flask containing the reactants was heated and the final temperature of the reaction solution reached 80°C, potassium carbonate ($K_2CO_3$; Sigma-Aldrich, ACS reagent) as a catalyst was added in an amount of about 0.05 g corresponding to a mole number that is 0.005 times one mole of the Raw material 1 to initiate the reaction. The transesterification reaction was performed for a total of 8 hours to obtain BHET, which is a value-added depolymerizable monomer. As the reaction proceeded, the produced methanol was degassed to be separated from the reactants by the flow of the carrier gas, discharged out of the reactor, and was collected by an external trap maintained at 0°C or below.

A small amount (less than 50 mg) of the liquid phase reactant was sampled at regular time intervals during the reaction, and the samples were quantified through high-performance liquid chromatography (HPLC with Optimapak C18 Column (250 mm, 5micron), UV detector ($\lambda$ = 254 nm)) calibrated in advance as a standard sample, the distribution and concentration of the reaction product were estimated from the results, and through this, and the conversion rate and yield of the reaction product were calculated. For the HPLC analysis, a mixed solution of methanol and water with a methanol:water volume ratio of 70:30 was used as the mobile phase, and the total flow rate was maintained at 0.7 mL/min.

The rate of conversion of DMT through transesterification is calculated by the following expression. In addition, the yields of the intermediate of partial transesterification of DMT using an alcohol reactant, the monomer product produced by complete transesterification, a dimer, and by-products are calculated by the following expressions. For example, the intermediate can be HEMT in the case of ethylene glycol addition transesterification, the monomer product can be BHET in the case of ethylene glycol addition transesterification, and the by-products can be potassium monomethyl terephthalate (K-MMT) and dipotassium terephthalate (K2-TPA).

$$\text{DMT conversion rate} = (N0 - N) / N0 \quad \text{(Expression 1)}$$

$$\text{Monomer yield} = (N_{monomer} / N0) \times 100\% \quad \text{(Expression 2)}$$

$$\text{Intermediate yield} = (\text{Nintermediate} / \text{N0}) \times 100\%$$

$$(\text{Expression 3})$$

$$\text{MMT yield} = (\text{NMMT} / \text{N0}) \times 100\% \quad (\text{Expression 4})$$

$$\text{TPA yield} = (\text{NTPA} / \text{N0}) \times 100\% \quad (\text{Expression 5})$$

$$\text{Timer yield} = (\text{Ndimer} / \text{N0}) \times 100\% \quad (\text{Expression 6})$$

In the above expressions, N0 is the number of moles of DMT, which is the initially fed raw material, and N, Nintermediate, Nmonomer, NMMT, NTPA, and Ndimer are the mole numbers of terephthalate contained in the unconverted DMT, intermediate, monomer product, MMT, TPA, and dimer.

Comparative Example 1

A transesterification reaction was carried out and evaluated in the same manner as in Example 1, except that potassium carbonate was not added unlike Example 1.

<Example 2>

A transesterification reaction was carried out and evaluated in the same manner as in Example 1, except that about 0.001 g potassium carbonate (corresponding to 0.0001 times the number of moles of Raw material 1) was used unlike Example 1.

<Example 3>

A transesterification reaction was carried out and evaluated in the same manner as in Example 1, except that about 0.01 g of potassium carbonate (corresponding to 0.001 times the number of moles of Raw material 1) was used in Example 1.

<Example 4>

A transesterification reaction was carried out and evaluated in the same manner as in Example 1, except that about 0.1 g of potassium carbonate (corresponding to 0.01 times the number of moles of Raw material 1) was used unlike Example 1.

<Example 5>

A transesterification reaction was carried out and evaluated in the same manner as in Example 1, except that about 0.5 g of potassium carbonate (corresponding to 0.05 times the number of moles of Raw material 1) was used unlike Example 1.

<Example 6>

A transesterification reaction was carried out and evaluated in the same manner as in Example 1, except that about 2.1 g of potassium carbonate (corresponding to 0.2 times the number of moles of Raw material 1) was used unlike Example 1.

Comparative Example 2

A transesterification reaction was carried out and evaluated in the same manner as in Example 1, except that the inert carrier gas was not used unlike Example 1.

<Example 7>

A transesterification reaction was carried out and evaluated in the same manner as in Example 1, except that the flow rate of high-purity nitrogen as the inert carrier gas was maintained at 50 sccm (GHSV=60h$^{-1}$) unlike Example 1.

<Example 8>

A transesterification reaction was carried out and evaluated in the same manner as in Example 1, except that the flow rate of high-purity nitrogen as the inert carrier gas was maintained at 100 sccm (GHSV=120h$^{-1}$) unlike Example 1.

<Example 9>

A transesterification reaction was carried out and evaluated in the same manner as in Example 1, except that the flow rate of high-purity nitrogen as the inert carrier gas was maintained at 500 sccm (GHSV=600h$^{-1}$) unlike Example 1.

<Example 10>

A transesterification reaction was carried out and evaluated in the same manner as in Example 1, except that the temperature of the reaction solution was maintained at 50°C unlike Example 1.

<Example 11>

A transesterification reaction was carried out and evaluated in the same manner as in Example 1, except that the temperature of the reaction solution was maintained at 65°C unlike Example 1.

<Example 12>

A transesterification reaction was carried out and evaluated in the same manner as in Example 1, except that the temperature of the reaction solution was maintained at 100°C unlike Example 1.

<Example 13>

A transesterification reaction was carried out and evaluated in the same manner as in Example 1, except that the temperature of the reaction solution was maintained at 110°C unlike Example 1.

<Example 14>

A transesterification reaction was carried out and evaluated in the same manner as in Example 1, except that the temperature of the reaction solution was maintained at 120°C unlike Example 1.

<Example 15>

A transesterification reaction was carried out and evaluated in the same manner as in Example 1, except that the temperature of the reaction solution was maintained at 130°C unlike Example 1.

<Example 16>

A transesterification reaction was carried out and evaluated in the same manner as in Example 1, except that the temperature of the reaction solution was maintained at 140°C unlike Example 1.

<Example 17>

A transesterification reaction was carried out and evaluated in the same manner as in Example 1, except that about 0.008 g of potassium bicarbonate (KHCO$_3$) (corresponding to 0.001 times the number of moles of Raw material 1) was used as a catalyst, and the reaction time was 3 hours unlike Example 1.

<Example 18>

A transesterification reaction was carried out and evaluated in the same manner as in Example 1, except that about 0.008 g of sodium carbonate ($Na_2CO_3$) (corresponding to 0.001 times the number of moles of Raw material 1) was used as a catalyst, and the reaction time was 3 hours unlike Example 1.

<Example 19>

A transesterification reaction was carried out and evaluated in the same manner as in Example 1, except that about 0.006 g of sodium bicarbonate ($NaHCO_3$) (corresponding to 0.001 times the number of moles of Raw material 1) was used as a catalyst, and the reaction time was 3 hours unlike Example 1.

<Comparative Example 3>

A transesterification reaction was carried out and evaluated in the same manner as in Example 1, except that about 0.007 g of potassium acetate (KOAc) (corresponding to 0.001 times the number of moles of Raw material 1) was used as a catalyst, and the reaction time was 3 hours unlike Example 1.

<Comparative Example 4>

A transesterification reaction was carried out and evaluated in the same manner as in Example 1, except that about 0.006 g of sodium acetate (NaOAc) (corresponding to 0.001 times the number of moles of Raw material 1) was used as a catalyst, and the reaction time was 3 hours unlike Example 1.

<Comparative Example 5>

A transesterification reaction was carried out and evaluated in the same manner as in Example 1, except that about 0.01 g of zinc acetate ($Zn(OAc)_2.2H_2O$) (corresponding to 0.001 times the number of moles of Raw material 1) was used as a catalyst, and the reaction time was 3 hours unlike Example 1.

<Example 20>

A transesterification reaction was carried out and evaluated in the same manner as in Example 1, except that about 0.004 g of potassium hydroxide (KOH) (corresponding to 0.001 times the number of moles of Raw material 1) was used as a catalyst, and the reaction time was 3 hours unlike Example 1.

<Example 21>

A transesterification reaction was carried out and evaluated in the same manner as in Example 1, except that about 0.003 g of sodium hydroxide (NaOH) (corresponding to 0.001 times the number of moles of Raw material 1) was used as a catalyst, and the reaction time was 3 hours unlike Example 1.

<Example 22>

A transesterification reaction was carried out and evaluated in the same manner as in Example 1, except that about 0.005 g of potassium methoxide ($CH_3OK$) (corresponding to 0.001 times the number of moles of Raw material 1) was used as a catalyst, and the reaction time was 3 hours unlike Example 1.

<Example 23>

A transesterification reaction was carried out and evaluated in the same manner as in Example 1, except that about 0.004 g of sodium methoxide ($CH_3ONa$) (corresponding to 0.001 times the number of moles of Raw material 1) was used as a catalyst, and the reaction time was 3 hours unlike Example 1.

<Example 24>

A transesterification reaction was carried out and evaluated in the same manner as in Example 1, except that about 0.003 g of magnesium oxide (MgO) (corresponding to 0.001 times the number of moles of Raw material 1) was used as a catalyst, and the reaction time was 3 hours unlike Example 1.

<Example 25>

A transesterification reaction was carried out and evaluated in the same manner as in Example 1, except that about 0.004 g of calcium oxide (CaO) (corresponding to 0.001 times the number of moles of Raw material 1) was used as a catalyst, and the reaction time was 3 hours unlike Example 1.

<Example 26>

A transesterification reaction was carried out and evaluated in the same manner as in Example 1, except that about 0.01 g of Triazabicyclodecene (TBD) (corresponding to 0.001 times the number of moles of Raw material 1) was used as a catalyst, and the reaction time was 3 hours unlike Example 1.

<Comparative Example 6: PET Depolymerization by High Temperature Glycolysis Reaction>

About 10 g of a PET raw material, Raw material 2, was prepared as a raw material for a glycolysis reaction. About 38.75 g of ethylene glycol as a dihydric alcohol polar solvent (i.e., 12 moles per mole of a polymer repeating unit) was put into a three-neck flask, a reflux condenser was installed at atmospheric pressure, and the materials in the flask were heated and stirred with a hot plate magnetic stirrer. When the temperature reached 177°C, 10 g of the prepared polymer raw material was added, and the heating and stirring were performed at the same time. When the reaction mixture reached 197°C or the reflux temperature, about 0.571 g of a zinc acetate catalyst (corresponding to 0.05 mole per mole of the polymer repeating unit) was added to initiate a catalytic reaction. The reaction mixture was continuously stirred for 2 hours during which the reaction temperature fluctuations were within $\pm 0.5$°C. For this reaction, a condenser with one end exposed to atmospheric pressure was used. Unreacted materials among the materials existing after the reaction products were separated and quantified through filtration. The monomers, dimers, and byproducts (monohydroxyethyl terephthalate (MHET) and oligomers) were quantified by high performance liquid chromatography calibrated in advance with a standard similar to the quantification of the reactants as in Example 1, and the distribution and concentration of the product were estimated.

<Example 27: PET Depolymerization by Room Temperature Methanolysis and Low Temperature Glycolysis>

About 3 g of PET raw material prepared according to Raw material 2, about 66.3 g of dichloromethane (Samchun Pure Chemical Co., Ltd.; purity 99.5%) (corresponding to 50 times the number of moles of the repeating unit of the polymer of Raw material 2), 24.96 g of methanol (Samchum Pure Chemical Co., Ltd.; 99.9%) (corresponding to 50 times the number of moles of the repeating unit of the polymer of Raw material 2), and about 0.43 g of potassium carbonate ($K_2CO_3$; Sigma-Aldrich, ACS reagent) (corresponding to 0.2 times the number of moles of the repeating unit of the polymer of Raw material 2) as a catalyst were put into a three-neck flask, and secondary distilled water was added to adjust the initial moisture content of the reactants. The initial moisture content was 0.4 mole per mole of the repeating unit of the raw material polymer. The reaction was carried out with stirring at 500 rpm for 24 hours at 25°C at atmospheric pressure using a magnetic stirrer.
After the reaction, the reaction mixture was filtered so as to be separated into filtrate and a filter cake. The filtrate included DMT, HEMT, ethylene glycol, and a portion of K-MMT, which is the potassium salt of MMT, and an organic solvent. The filter cake contained the remaining unreacted raw materials, such as PET, $K_2CO_3$ catalyst, and K-MMT.
Each of the filtrate and the potassium salt in the filter cake was taken in a trace amount (less than 50 mg), and then diluted in an aqueous mobile phase solution to prepare samples. The product distribution and concentration in each sample were estimated through quantitative analysis using high-performance liquid chromatography (HPLC with Optimapak C18 Column (250 mm, 5micron), UV detector ($\lambda$=254nm)), and the unreacted PET in the filter cake was quantified gravimetrically. On the basis of the quantified values, the conversion of PET and the yields of DMT, HEMT, TPA, and MMT according to the depolymerization reaction were calculated. For the HPLC analysis, a mixed solution of methanol and water with a volume ratio of 70:30 was used as the mobile phase, and the total flow rate was maintained at 0.7 mL/min.
The conversion rate of PET and the yield of DMT by the depolymerization reaction were calculated by the following expressions.

```
PET conversion rate = (M0 − M) / M0 × 100%   (Expression 7)
```

$$\mathrm{DMT\ yield = (MDMT\ /\ M0) \times 100\%}\quad\text{(Expression 8)}$$

In the above expressions, M0 and M represent the number of moles of repeating units of the initially introduced raw material polymer (PET) and the unreacted polymer, respectively, and MDMT represents the number of moles of DMT produced.

The filtrate of the reaction solution of the depolymerization was used as a raw material for a transesterification reaction in which ethylene glycol was added. The transesterification reaction was carried out under the same conditions as in Example 1, but the filtrate of the depolymerization product was used instead of Raw material 1 as a raw material, and most of the methanol and polar aprotic solvent were removed using a vacuum evaporator. Thereafter, the number of moles of DMT was adjusted to be the same as in Example 1, the total amount of ethylene glycol including ethylene glycol produced by the depolymerization and newly added ethylene glycol was about 55.6 g (corresponding to 12 times the number of moles of DMT), and no catalyst was used. Under these conditions, a transesterification reaction and evaluation were performed.

The number of moles of the catalyst contained in the recovered filter cake and the number of moles of K-MMT were quantified using the gravimetric method and HPLC, and determined to be 0.126 and 0.024 moles per mole of the repeating unit of the raw material, respectively. The number of moles of the catalyst (based on the mole balance of potassium cations) existing in the filtrate of the depolymerization reaction solution was calculated by subtracting the number of moles of the catalyst contained in the recovered filter cake from the number of moles of the initially introduced catalyst. From this estimation, it was found that the amount of catalyst ($K_2CO_3$) existed in an amount of 0.054 moles per mole of DMT of the raw materials of the transesterification reaction performed with the addition of ethylene glycol.

The conversion rate of DMT and the yields of BHET, HEMT, and BHET dimer by the transesterification reaction were calculated using Expressions 1 to 4, and the results are shown in Table 5.

<Example 28>

A transesterification reaction was carried out and evaluated in the same manner as in Example 1, except that instead of ethylene glycol as a reactant, the same moles (12 times the number of moles of DMT) of 1,3-propanediol (Sigma-Aldrich; purity ≥ 98%) was used, and the temperature of the reaction solution was maintained at 100°C.

<Example 29>

A transesterification reaction was carried out and evaluated in the same manner as in Example 1, except that instead of ethylene glycol as a reactant, the same moles (12 times the number of moles of DMT) of 1,4-butanediol (Sigma-Aldrich; ReagentPlus, purity ≥ 99%) was used, and the temperature of the reaction solution was maintained at 100°C.

<Example 30>

A transesterification reaction was carried out and evaluated in the same manner as in Example 1, except that instead of ethylene glycol as a reactant, the same moles (12 times the number of moles of DMT) of 1,6-hexanediol (Sigma-Aldrich; purity ≥ 99%) was used, and the temperature of the reaction solution was maintained at 100°C.

<Example 31>

A transesterification reaction was carried out and evaluated in the same manner as in Example 1, except that instead of ethylene glycol as a reactant, the same moles (12 times the number of moles of DMT) of 1,2-propanediol (Sigma-Aldrich; ReagentPlus, purity ≥ 99%) was used, and the temperature of the reaction solution was maintained at 100°C.

<Example 32>

A transesterification reaction was carried out and evaluated in the same manner as in Example 1, except that instead of ethylene glycol as a reactant, the same moles (12 times the number of moles of DMT) of 1,3-butanediol (Sigma-Aldrich; Anhyrous, purity ≥ 99%) was used, and the temperature of the reaction solution was maintained at 100°C.

<Example 33>

A transesterification reaction was carried out and evaluated in the same manner as in Example 1, except that instead of ethylene glycol as a reactant, the same moles (12 times the number of moles of DMT) of 1,4-cyclohexanedimethanol (Sigma-Aldrich; mixture of cis and trans, purity ≥ 99%) was used, and the temperature of the reaction solution was maintained at 100°C.

<Example 34>

A transesterification reaction was carried out and evaluated in the same manner as in Example 1, except that instead of ethylene glycol as a reactant, the same moles (12 times the number of moles of DMT) of 2-ethyl-1-hexanol (Sigma-Aldrich; purity ≥ 99.6%) was used, and the temperature of the reaction solution was maintained at 100°C.

[Comparison of Transesterification Reaction Characteristics according to Amount of Catalyst]

Table 1 shows the result of observing the effect of the reaction while adjusting the amount of potassium carbonate ($K_2CO_3$) used as a catalyst according to the examples of the present disclosure in carrying out the transesterification reaction in Examples 1 to 6 and Comparative Example 1 (reaction temperature 80°C, GHSV = 240 $h^{-1}$). Unlike the examples in which the catalyst was used, in the case of Comparative Example 1 in which no catalyst was used, the yields of BHET were very low to be 0.0% and 2.4% after 2 hours and 8 hours, respectively. The results explain that the use of a catalyst is essential in promoting the low-temperature transesterification reaction of DMT with ethylene glycol according to the present disclosure.

[Table 1]

| classification | Reaction Time (h) | number of moles of added compound per 1 mol of DMT | | Conversion Rate (%) | Yield (%) | | | | | |
|---|---|---|---|---|---|---|---|---|---|---|
| | | EG | $K_2CO_3$ | | BHET | HEMT | BHET dimer | $K_2$-TPA | K-MMT | oligomer |
| Comparative Example 1 | 2 | 12 | 0.0 | 2.2 | 0.0 | 1.7 | 0.0 | 0.0 | 0.0 | 0.0 |
| | 8 | 12 | 0.0 | 8.9 | 2.4 | 6.5 | 0.0 | 0.0 | 0.0 | 0.0 |
| Example 2 | 2 | 12 | 0.0001 | 64.5 | 43.8 | 20.2 | 0.4 | 0.0 | 0.0 | 0.0 |
| | 8 | 12 | 0.0001 | 100.0 | 81.9 | 16.6 | 1.5 | 0.0 | 0.0 | 0.0 |
| Example 3 | 2 | 12 | 0.001 | 99.5 | 85.7 | 12.1 | 1.7 | 0.0 | 0.0 | 0.0 |
| | 8 | 12 | 0.001 | 100.0 | 97.2 | 0.6 | 2.2 | 0.0 | 0.0 | 0.0 |
| Example 1 | 2 | 12 | 0.005 | 99.8 | 86.3 | 8.6 | 1.7 | 0.7 | 0.0 | 0.0 |
| | 8 | 12 | 0.005 | 100.0 | 97.0 | 0.0 | 2.1 | 0.9 | 0.0 | 0.0 |
| Example 4 | 2 | 12 | 0.01 | 100.0 | 88.2 | 9.0 | 1.8 | 1.0 | 0.0 | 0.0 |
| | 8 | 12 | 0.01 | 100.0 | 96.6 | 0.0 | 2.2 | 1.2 | 0.0 | 0.0 |
| Example 5 | 2 | 12 | 0.05 | 99.5 | 80.9 | 11.6 | 1.4 | 5.6 | 0.0 | 0.0 |
| | 8 | 12 | 0.05 | 99.8 | 84.0 | 8.2 | 1.6 | 6.0 | 0.0 | 0.0 |
| Example 6 | 2 | 12 | 0.2 | 99.3 | 67.4 | 11.6 | 0.9 | 17.4 | 2.0 | 0.0 |
| | 8 | 12 | 0.2 | 99.1 | 65.7 | 13.2 | 0.8 | 18.5 | 0.9 | 0.0 |

DMT: Dimethyl terephthalate, EG: Ethylene glycol,
BHET: Bis(2-hydroxyethyl) terephthalate,
HEMT: 1-(2-Hydroxyethyl) 4-methyl terephthalate
$K_2$-TPA: Dipotassium terephthalate
K-MMT: Potassium monomethyl terephthalate

Examples 1 to 4 are the results of the reactions using, as the catalyst, 0.0001 to 0.01 mol of potassium carbonate per 1 mole of DMT, which is Raw material 1. It is found that the production rate and yield of BHET increases with an increase in the amount of potassium carbonate.

On the other hand, in Examples 5 and 6, 0.05 mol and 0.2 mol of potassium carbonate were used per 1 mol of DMT, respectively. In these examples, the production rate and yield of BHET decreased. However, in Examples 5 and 6, the final yield of K2-TPA, which is a by-product of the reaction, was increased to 6.0% and 18.5%, respectively. These results show that side reactions such as hydrolysis or alkali decomposition as well as catalyst consumption are promoted when the number of moles of the catalyst is excessive relative to the number of moles of DMT injected. Therefore, the appropriate control of the amount of catalyst can help improve the performance and economics of transesterification.

[Comparison of Transesterification Characteristics According to Gas Space Velocity (GHSV)]

When a transesterification reaction of DMT is performed in the presence of an excessive amount of an ethylene glycol solvent, the production rate of BHET, which is a reaction product, is observed to be very high at the beginning of the reaction, and the production rate of methanol, which is a by-product of the transesterification reaction, increases in proportional to the number of moles of BHET produced. In a closed system, this transesterification reaction is not an irreversible reaction in which the entire DMT introduced initially is converted to BHET, and a reverse transesterification reaction in which the methanol byproduct and BHET participate may proceed at a certain speed. When the forward reaction and the reverse reaction reach equilibrium, the concentration of the compound no longer changes, and even through the reaction conditions are changed in such a condition, there may be a limit to improving the yield of the product. However, when a part of the reaction product is effectively removed from the reaction product mixture or discharged to the outside of the reaction system, the reverse reaction can be suppressed, and the characteristics of the reaction can be determined by the concentration change of the substance, that is, the mass transfer rate of the substance discharged to the outside.

Methanol produced from the transesterification reaction performed according to an example of the present disclosure is a substance with the lowest boiling point (130°C or lower than that of ethylene glycol) among the added raw materials and the produced reaction products. The methanol can be selectively discharged to the outside of the reaction system by increasing the temperature of the reaction system, lowering the pressure of the reaction, or making a continuous flow of a carrier gas through the reaction system. With this measure, a reverse transesterification reaction of generating DMT can be inhibited.

[Table 2]

| classification | Reaction Time (h) | number of moles of added compound per 1 mol of DMT | | GHSV ($h^{-1}$) | Conversion Rate (%) | Yield (%) | | | | | |
|---|---|---|---|---|---|---|---|---|---|---|---|
| | | EG | $K_2CO_3$ | | | BHET | HEMT | BHET dimer | $K_2$-TPA | K-MMT | oligomer |
| Comparative Example 2 | 3 | 12 | 0.005 | 0 | 88.9 | 58.4 | 29.5 | 0.8 | 0.3 | 0.0 | 0.0 |
| | 8 | 12 | 0.005 | 0 | 90.0 | 59.3 | 29.5 | 0.8 | 0.4 | 0.0 | 0.0 |
| Example 7 | 3 | 12 | 0.005 | 60 | 98.5 | 77.7 | 19.0 | 1.3 | 0.5 | 0.0 | 0.0 |
| | 8 | 12 | 0.005 | 60 | 100.0 | 89.9 | 7.6 | 1.8 | 0.7 | 0.0 | 0.0 |
| Example 8 | 3 | 12 | 0.005 | 120 | 98.9 | 79.7 | 17.3 | 1.3 | 0.7 | 0.0 | 0.0 |
| | 8 | 12 | 0.005 | 120 | 100.0 | 94.6 | 2.4 | 1.9 | 1.1 | 0.0 | 0.0 |
| Example 1 | 3 | 12 | 0.005 | 240 | 100.0 | 92.6 | 4.2 | 1.9 | 0.8 | 0.0 | 0.0 |
| | 8 | 12 | 0.005 | 240 | 100.0 | 97.0 | 0.0 | 2.1 | 0.9 | 0.0 | 0.0 |
| Example 9 | 3 | 12 | 0.005 | 600 | 100.0 | 97.0 | 0.3 | 2.1 | 0.6 | 0.0 | 0.0 |
| | 8 | 12 | 0.005 | 600 | 100.0 | 83.7 | 0.0 | 1.7 | 0.6 | 0.0 | 13.9 |
| DMT: Dimethyl terephthalate, EG: Ethylene glycol, BHET: Bis(2-hydroxyethyl) terephthalate, HEMT: 1-(2-Hydroxyethyl) 4-methyl terephthalate $K_2$-TPA: Dipotassium terephthalate K-MMT: Potassium monomethyl terephthalate | | | | | | | | | | | |

Table 2 is the results of observation for the characteristics of the transesterification reaction performed by varying the flow rate of an inert carrier gas (high purity nitrogen) that is introduced into the reactor to cause gas-liquid contact with the reactants inside the reactor to separate methanol from the reaction products (reaction temperature 80°C).

When there is no flow of a carrier gas (Comparative Example 2), as a result of transesterification at 80°C for 3 hours, a relatively low yield (58.4%) of BHET was observed. When the reaction was performed for a relatively long time of about 8 hours, only an insignificant increase of about 0.9% in the reaction yield was observed. Although the reactor is in the form of an open system and the internal temperature is maintained above the boiling point of methanol, when the transesterification proceeds partially, dominant diffusion of the produced methanol into a head-space which is not filled with reaction products or into the external environment outside the reactor, and it is assumed that the methanol is present in a high concentration among the reaction products.

On the other hand, in the case of transesterification reactions performed under continuous flow conditions with different flow rates of nitrogen as a carrier gas (Example 1 and Examples 7 to 9), it is confirmed that the conversion rate and the yield of BHET are greatly improved. In particular, in the reactions carried out according to Examples 1 and 9 in which the GHSV exceeds 200, it is confirmed that the yield of BHET yield is 92% or more when the reaction time exceeds 3 hours.

On the other hand, it was confirmed that the BHET yield was not improved because the yield of dimers and oligomers slightly increased when the flow rate of the carrier gas was excessively high, and the reaction time was excessively long. In the case of Example 1, even though the reaction was performed for 3 hours or more, oligomers were not observed among the reactants. On the other hand, when the transesterification reaction was performed according to Example 9, it was observed that the selectivity for BHET decreased as oligomers started to be produced after 6 hours. Even though only a small amount of oligomer, less than 3%, was produced, the viscosity of the reaction mixture greatly increased, and a phenomenon in which a portion of the reaction product forms a non-uniform phase, and the reactants were rapidly suspended was observed. Therefore, in order to maximize the yield of BHET, which is a monomer produced through a polymer depolymerization, a carrier gas flow is applied. In this case, to improve the yield of the end product, BHET, it is necessary to select the optimum carrier gas conditions under which methanol can be efficiently removed, and an ethylene glycol addition reaction can predominantly occur.

[Change of Characteristics of Transesterification Reaction Depending on Reaction Temperature]

Table 3 shows the result of observation of a transesterification reaction of DMT with ethylene glycol while varying the reaction temperature (GHSV = $240h^{-1}$).

When maintaining the temperature above the boiling point of methanol (Example 1 and Examples 12 to 16), the yield of BHET obtained from the transesterification reaction could be maintained high from the beginning of the reaction. When the reaction time exceeded 4 hours, the yield of BHET was 95% or more. This improvement in yield is attributed to the increase in the mass transfer rate of methanol when the reaction temperature exceeds the boiling point of methanol. The improvement in yield is also attributed to the increase in the catalytic reaction rate of sequential glycolysis reactions in which DMT is converted to HEMT and HEMT is then converted to BEHT.

[Table 3]

| classification | Reaction Time (h) | number of moles of added compound per 1 mol of DMT | | Reaction Temperature (°C) | Conversion Rate (%) | Yield (%) | | | | | |
|---|---|---|---|---|---|---|---|---|---|---|---|
| | | EG | $K_2CO_3$ | | | BHET | HEMT | BHET dimer | $K_2$-TPA | K-MMT | oligomer |
| Example 10 | 2 | 12 | 0.005 | 50 | 58.4 | 45.4 | 12.5 | 0.4 | 0.0 | 0.0 | 0.0 |
| | 4 | 12 | 0.005 | 50 | 88.1 | 68.1 | 19.1 | 0.9 | 0.0 | 0.0 | 0.0 |
| Example 11 | 2 | 12 | 0.005 | 65 | 93.7 | 73.9 | 18.3 | 1.2 | 0.4 | 0.0 | 0.0 |
| | 4 | 12 | 0.005 | 65 | 97.5 | 86.8 | 8.4 | 1.6 | 0.7 | 0.0 | 0.0 |
| Example 1 | 2 | 12 | 0.005 | 80 | 99.8 | 86.3 | 8.6 | 1.7 | 0.7 | 0.0 | 0.0 |
| | 4 | 12 | 0.005 | 80 | 100.0 | 95.6 | 1.7 | 2.1 | 0.6 | 0.0 | 0.0 |
| Example 12 | 2 | 12 | 0.005 | 100 | 100.0 | 95.8 | 1.5 | 2.2 | 0.5 | 0.0 | 0.0 |
| | 4 | 12 | 0.005 | 100 | 100.0 | 97.1 | 0.0 | 2.3 | 0.6 | 0.0 | 0.0 |
| Example 13 | 2 | 12 | 0.005 | 110 | 100.0 | 95.4 | 0.6 | 2.2 | 0.9 | 0.0 | 0.0 |
| | 4 | 12 | 0.005 | 110 | 100.0 | 96.6 | 0.3 | 2.2 | 0.9 | 0.0 | 0.0 |
| Example 14 | 2 | 12 | 0.005 | 120 | 100.0 | 92.6 | 4.6 | 2.0 | 0.8 | 0.0 | 0.0 |
| | 4 | 12 | 0.005 | 120 | 100.0 | 95.0 | 1.8 | 2.2 | 1.0 | 0.0 | 0.0 |
| Example 15 | 2 | 12 | 0.005 | 130 | 100.0 | 96.5 | 0.3 | 2.4 | 0.8 | 0.0 | 0.0 |
| | 4 | 12 | 0.005 | 130 | 100.0 | 96.6 | 0.0 | 2.4 | 1.0 | 0.0 | 0.0 |
| Example 16 | 2 | 12 | 0.005 | 140 | 100.0 | 95.1 | 0.7 | 2.4 | 0.8 | 0.0 | 0.0 |
| | 4 | 12 | 0.005 | 140 | 100.0 | 96.4 | 0.0 | 2.4 | 0.9 | 0.0 | 0.0 |

DMT: Dimethyl terephthalate, EG: Ethylene gycol,
BHET: Bis(2-hydroxyethyl) terephthalate,
HEMT: 1-(2-Hydroxyethyl) 4-methyl terephthalate
$K_2$-TPA: Dipotassium terephthalate
K-MMT: Potassium monomethyl terephthalate

On the other hand, when the transesterification reaction was carried out at the reaction temperature below the boiling point of methanol (Example 10 and Example 11), the yield of BHET according to the reaction time was 45.4% and 73.9% after 2 hours, respectively in Example 10 and Example 11, and 68.1% and 86.8% after 4 hours, respectively in Example 10 and Example 11.

In addition, although not shown in the table, oligomers began to be detected as they were exposed to reaction conditions for a long time (after 7 hours of the reaction). After 8 hours of the reaction, about 11.2% and 3.3% of the applied DMT, which is a raw material, were converted into oligomers. In this case, the viscosity of the reaction solution greatly increased, and it was also observed that the reaction solution changed to a non-uniform suspension.

In summary, it was found that it is more advantageous to control the reaction temperature to be above the boiling point of methanol in order to increase the conversion of DMT within a short reaction time or retention time and to improve the yield of BHET, which is a monomer, without formation of oligomers.

[Comparison of Transesterification Reaction Characteristics according to Selection of Reaction Catalyst]

Existing glycolysis reactions for producing BHET from polymers containing ester functional groups are performed at high temperatures, so a large amount of energy consumption is pointed out as a problem. In order to overcome this problem, a novel reaction pathway capable of producing BHET with high yield in a low temperature region can be considered.

When, as an example of such a reaction pathway, the methanolysis reaction for producing DMT from a polymer raw material containing an ester functional group can be performed at room temperature, and a low-temperature reaction condition in which ethylene glycol addition can occur continuously can be provided, it can be expected that BHET can be produced in high yield.

However, it is necessary to design or develop a new catalyst system that may partially remain in the reaction mixture when an additional separation process is not performed after methanolysis (primary reaction), but the remaining catalyst can exhibit a sufficient catalytic activity in the subsequent transesterification reaction.

Catalysts commonly used in transesterification reactions may include acids, bases, and metal salts. Since acidic catalysts exhibit a slow reaction rate than basic catalysts, the acidic catalysts are commonly used for reaction at high temperature (above 100°C). In this case, to prevent corrosion, an acid-resistant design for the reactor and auxiliary facilities is required, which may result in excessive initial investment.

Therefore, in the present disclosure, for basic catalysts and metal salt catalysts, which are expected to exhibit reactivity and selectivity for the ethylene glycol addition transesterification reaction of DMT, the reaction performance evaluation and usefulness have been studied.

In order to compare the relative performance of each catalyst, the same reaction conditions were set except for the type of catalyst. That is, the input amount of the ester reaction catalyst was 0.001 mol per 1 mol of DMT, the flow rate of a carrier gas was, GHSV 240 $h^{-1}$, the reaction temperature was 80°C, and the reaction time was 3 hrs.

As catalysts for performance evaluation, acetic acid metal salts (for example, zinc acetate), alkali carbonates, alkali hydroxides, alkali alkoxides, alkaline earth metal oxides, and guanidine-based organic compounds, which are expected to exhibit the best performance in the transesterification reaction, were selected and used. Their reaction performance and acid dissociation equilibrium constant (pKa) are shown in [Table 4] (reaction temperature 80°C, reaction time 3 hrs, GHSV = 240 $h^{-1}$).

[Table 4]

| classification | Type of catalyst | number of moles of added compound per 1 mol of DMT | | Conversion Rate (%) | Yield (%) | | | | | | pKa |
|---|---|---|---|---|---|---|---|---|---|---|---|
| | | EG | Catalyst | | BHET | HEMT | BHET dimer | $K_2$-TPA | K-MMT | oligomer | |
| Comp. Ex. 3 | KOAc | 12 | 0.001 | 9.8 | 3.1 | 6.7 | 0.0 | 0.0 | 0.0 | 0.0 | 4.8 |
| Comp. Ex. 4 | NaOAc | 12 | 0.001 | 10.7 | 3.6 | 7.1 | 0.0 | 0.0 | 0.0 | 0.0 | 4.8 |
| Comp. Ex. 5 | Zn(OAc)$_2$ | 12 | 0.001 | 4.0 | 0.7 | 3.3 | 0.0 | 0.0 | 0.0 | 0.0 | 4.5 |
| Example 3 | $K_2CO_3$ | 12 | 0.001 | 100.0 | 92.0 | 6.0 | 2.0 | 0.0 | 0.0 | 0.0 | 10.3 |
| Example 17 | $KHCO_3$ | 12 | 0.001 | 98.5 | 76.8 | 20.4 | 1.3 | 0.0 | 0.0 | 0.0 | 6.4 |
| Example 18 | $Na_2CO_3$ | 12 | 0.001 | 100.0 | 92.3 | 5.7 | 2.0 | 0.0 | 0.0 | 0.0 | 10.3 |
| Example 19 | $NaHCO_3$ | 12 | 0.001 | 98.4 | 78.9 | 18.2 | 1.4 | 0.0 | 0.0 | 0.0 | 6.4 |
| Example 20 | KOH | 12 | 0.001 | 100.0 | 91.4 | 6.7 | 1.9 | 0.0 | 0.0 | 0.0 | 15.7 |
| Example 21 | NaOH | 12 | 0.001 | 100.0 | 91.9 | 6.2 | 1.9 | 0.0 | 0.0 | 0.0 | 15.7 |
| Example 22 | $CH_3OK$ | 12 | 0.001 | 100.0 | 90.6 | 7.5 | 1.9 | 0.0 | 0.0 | 0.0 | 22.1 |
| Example 23 | $CH_3ONa$ | 12 | 0.001 | 100.0 | 83.6 | 14.7 | 1.7 | 0.0 | 0.0 | 0.0 | 22.1 |
| Example 24 | MgO | 12 | 0.001 | 96.0 | 77.4 | 17.3 | 1.3 | 0.0 | 0.0 | 0.0 | 15.7 |
| Example 25 | CaO | 12 | 0.001 | 100.0 | 90.6 | 7.5 | 1.9 | 0.0 | 0.0 | 0.0 | 12.8 |
| Example 26 | TBD | 12 | 0.001 | 100.0 | 90.5 | 7.6 | 1.9 | 0.0 | 0.0 | 0.0 | 15.2 |

DMT: Dimethyl terephthalate, EG: Ethylene glycol,
BHET: Bis(2-hydroxyethyl) terephthalate,
HEMT: 1-(2-Hydroxyethyl) 4-methyl terephthalate,
KOAc : $CH_3COOK$, NaOAc : $CH_3COONa$, Zn(OAc)$_2$ : Zn($CH_3COO$)·2H$_2$O,
TBD: Triazabicyclodecene
$K_2$-TPA: Dipotassium terephthalate
K-MMT: Potassium monomethyl terephthalate

Metal salts of acetic acid are used as catalysts in a general high-temperature reaction process for producing BHET through depolymerization of a polymer having an ester functional group. In particular, zinc acetate $(Zn(CH_3COO)_2 \cdot 2H_2O)$ is most commonly used in commercial processes because of its high polymer depolymerization reactivity and excellent selectivity to BHET. In order to confirm the catalytic effectiveness of metal salts of acetic acid for a transesterification reaction according to the present disclosure, potassium acetate, sodium acetate, and zinc acetate were added as catalysts, and then reaction experiments were performed.

In the case of Comparative Examples 3 and 4 in which potassium acetate and sodium acetate were used as catalysts under the same conditions, very low reaction performance was observed with a BHET yield of 4% or less after 3 hours of the reaction. In addition, in the case of Comparative Example 5 using zinc acetate as a catalyst, the yield of BHET was 0.7%. That is, almost no BHET was produced, similar to the case of Comparative Example 1 in which not catalyst was used. Therefore, it was confirmed that metal salts of acetic acid, which can be usefully used for conventional high-temperature ($\geq 190°C$) glycolysis reactions, cannot be used as efficient catalysts for the transesterification reaction performed according to the present disclosure.

The reaction results of Example 3 and Examples 17 to 19 using a metal salt catalyst composed of carbonic acid and bicarbonate as anions were compared. In the case where the reaction was performed for 3 hours using potassium carbonate ($K_2CO_3$) and sodium carbonate ($Na_2CO_3$) as catalysts, the yields of BHET were 92.0% and 92.3%, respectively, which were about 13% higher than that of the case where potassium bicarbonate ($KHCO_3$) and sodium bicarbonate ($NaHCO_3$) were used as catalysts.

An alkali hydroxide (or alkali metal hydroxide) is used as a representative raw material for an alkali decomposition reaction among depolymerization reactions of polymers containing ester functional groups. In the alkali decomposition reaction, since the alkali hydroxide directly participates as a reactant in the breaking of ester bonds, it is supplied in an excessive amount compared to the number of moles of ester bond groups in the polymer, and water or alcohol is used as a medium for the reaction. Ethylene glycol is produced as a reaction product.

In the present application, the function of alkali hydroxide as a catalyst rather than a reactant for alkali decomposition was observed. Under the reaction conditions according to the present application, that is, when ethylene glycol is supplied in excess, a small amount of alkali hydroxide is added, and the reaction temperature is between room temperature (25°C.) and the boiling point of ethylene glycol, the function as a catalyst for transesterification can be exhibited. Potassium hydroxide and sodium hydroxide (Examples 20 and 21) were added in small amounts (i.e., 0.001 mol per 1 mol of DMT), and transesterification was performed. As a result, the entire DMT was converted, and the BHET yield after 3 hours of the reaction was 91% or more.

Next, for the case where an alkoxide metal salt was used as an alternative catalyst, the performance of the transesterification reaction was confirmed. When the reaction was performed with the use of potassium methoxide ($CH_3OK$) or sodium methoxide ($CH_3ONa$) as a catalyst (Examples 22 or 23), high reactivity was observed, and the BHET yields after 3 hours of the reaction were as high as 90.6% and 83.6%, respectively. These catalysts were effective in enhancing the rate of the primary-stage ethylene glycol addition reaction (HEMT production) of DMT compared to the case where alkali metal-based carbonate catalysts were used (Examples 3 and 18), but exhibited relatively slightly low reaction rate in the secondary addition (BHET production).

High performance of the transesterification reaction was also observed when an alkaline earth metal-based oxide catalyst was applied. As a result of reactions in which magnesium oxide and calcium oxide were used as catalysts (Examples 24 and 25), the conversion rate was observed to be 96% or more, and the yields of BHET after 3 hours of the reaction was 77.4% and 90.6%, respectively, in Examples 24 and 25.

Finally, the reaction characteristics of metal-free organic catalysts were compared. In Example 26, triazabicyclodecene (TBD), which is a guanidine-based compound that has strong basic properties and is commonly used as a catalyst for organic compound synthesis, was used as a catalyst for the transesterification reaction. Similar to the case of Example 3 using potassium carbonate ($K_2CO_3$) as a catalyst, almost the entire DMT added after 3 hours of the reaction was involved in the glycolysis reaction, and the yield of BHET was 90.5% which is relatively high.

The reaction performance according to the acid dissociation equilibrium constant (pKa) of each catalysts used was compared as in Table 4. In order to obtain a high yield of BHET by performing the low temperature ($\leq 100°C$) glycolysis reaction according to the present disclosure, it was confirmed the suitable catalyst had a pKa value of 6 or more.

[Production of BHET by Transesterification of Polymer Containing Ester Functional Group]

Glycolysis reactions for directly producing BHET by depolymerization of a polymer containing an ester functional group are generally carried out at a high temperature in a range of 190°C to 300°C. As reported in a number of literatures, high-temperature glycolysis reactions are commonly performed at temperatures close to or higher than the boiling point of ethylene glycol, which is an additive reactant, using a metal acetate as a catalyst. In this catalytic reaction, the yield of the monomer product (BHET) of the glycolysis is 80% or more which is higher than that of other compounds, but it was observed that by-products (for example, monohydroxyethyl terephthalate (MHET), and terephthalic acid), were generated from side reactions such as hydrolysis, and dimers or oligomers were formed at a certain rate according to the reaction equilibrium. On the other hand, when depolymerization is performed by a low-temperature methanolysis-glycolysis tandem reaction pathway with different temperature ranges and BHET production mechanisms according to an example of the present disclosure, high-purity BHET containing only small amounts of reactive intermediates (for example, HEMT) and dimers can be obtained in high yield.

Table 5 shows the comparison results of high-temperature (190°C) transesterification reaction and low-temperature transesterification reaction (25°C methanolysis and 80°C ethylene glycol addition transesterification) in each of which the same raw material, Raw material 2, was used.

In the table below, the number of moles of each of EG and catalyst in Comparative Example 6 is based on the repeating unit of PET, and the number of moles of each of EG and catalyst in Example 27 is based on DMT.

[Table 5]

| Classification (Catalyst) | Reaction Temperature (°C) | Reaction Time (h) | Number of moles of added compound per 1 mol of DMT or repeating unit of PET | | Conversion Rate (%) | Yield (%) | | | | | |
|---|---|---|---|---|---|---|---|---|---|---|---|
| | | | EG | Catalyst | | BHET | MHET | HEMT | BHET dimer | $K_2$-TPA/ K-MMT | oligomer |
| Comp. Ex. 6 ($Zn(OAc)_2$) | 190 | 2 | 12 | 0.05 | 100.0 | 80.6 | 2.2 | - | 4.1 | - | 13.1 |
| | | 8 | 12 | 0.05 | 100.0 | 82.1 | 3.5 | - | 4.1 | - | 10,3 |
| Example 27 ($K_2CO_3$) | 80 | 2 | 12 | 0.05 | 100.0 | 90.6 | - | 2.9 | 2.3 | 4.2/0.0 | 0.0 |
| | | 8 | 12 | 0.05 | 100.0 | 92.3 | - | 0.4 | 2.6 | 4.7/0.0 | 0.0 |

DMT: Dimethyl terephthalate, EG: Ethylene glycol,
BHET: Bis(2-hydroxyethyl) terephthalate,
HEMT: 1-(2-Hydroxyethyl) 4-methyl terephtnalate
MHET: mono(hydroxyethyl) terephthalate
$K_2$-TPA: Dipotassium terephthalate
K-MMT: Potassium monomethyl terephthalate

When a depolymerization reaction was carried out at high temperature using zinc acetate (Comparative Example 6), polymer decomposition occurred rapidly from the beginning of the reaction (within 2 hours), and the yield of BHET was 80.6%. However, when exposed to the predetermined reaction conditions for a relatively long time (about 8 hours), the increase in the yield of BHET was not remarkable, the yield of dimers was about 4.1%, and the yield of oligomers was about 10% to 15%. That is, the concentration of undecomposed compounds was high.

In Example 27, a methanolysis-based room temperature (25°C) depolymerization was performed using the same PET (Raw material 2) as in the previous case as a raw material, and a co-solvent to obtain a liquid reaction mixture (DMT yield: 91%). The liquid reaction mixture was filtered to remove a portion of the catalyst, followed by heating to remove the solvent. After that, a glycolysis (secondary reaction) was performed by adding ethylene glycol. In the case of this reaction, reaction characteristics and product distribution very different from those of the high-temperature glycolysis of Comparative Example 6 were observed.

In Example 27, a very high DMT conversion rate was observed from the beginning of the reaction, and a BHET yield exceeding 92% was observed when the reaction solution was left in the reaction conditions for a sufficient period of time (8 hours or more). The distribution of reaction by-products was observed. MHET was obtained as a reaction by-product in the high-temperature glycolysis of Comparative Example 6, but in Example 27, HEMT in which ethylene glycol was added at one side was obtained as a reaction intermediate, and the reaction proceeded relatively quickly. It was observed that only a very low HEMT concentration of less than 0.5% remained after 8 hours of the reaction.

From the results, it can be confirmed that in preparing BHET from a polymer containing an ester functional group through a low-temperature methanolysis-glycolysis reaction, the same catalyst can be used continuously for each reaction.

[Comparison of Characteristics of Transesterification Reaction and Yield of Monomer products by Type of Alcohol Reactant]

Table 6 shows the results of observing the transesterification reaction characteristics performed by adding different types of monohydric or polyhydric alcohols instead of ethylene glycol, which is a raw material for the glycolysis reaction, when preparing a monomer from a polymer containing an ester functional group ( Reaction temperature 100°C, GHSV=240h$^{-1}$).

[Table 6]

| Classification (Reactant used) | Reaction Time (h) | number of moles of added compound per 1 mol of DMT | | Conversion Rate (%) | Yield (%) | | | | | |
| | | Alcohol Reactant | Catalyst | | Monomer Product | Intermediate | Dimer | $K_2$-TPA | K-MMT | oligomer |
|---|---|---|---|---|---|---|---|---|---|---|
| Example 12 (EG) | 2 | 12 | 0.005 | 100.0 | 95.8 | 1.5 | 2.2 | 0.5 | 0.0 | 0.0 |
| | 8 | 12 | 0.005 | 100.0 | 96.9 | 0.0 | 2.3 | 0.8 | 0.0 | 0.0 |
| Example 28 (1,3-PDO) | 2 | 12 | 0.005 | 100.0 | 89.7 | 6.2 | 3.6 | 0.4 | 0.0 | 0.0 |
| | 8 | 12 | 0.005 | 100.0 | 93.2 | 2.2 | 4.0 | 0.5 | 0.0 | 0.0 |
| Example 29 (1,4-BDO) | 2 | 12 | 0.005 | 97.3 | 92.6 | 0.4 | 3.8 | 0.6 | 0.0 | 0.0 |
| | 8 | 12 | 0.005 | 100.0 | 95.5 | 0.0 | 3.9 | 0.6 | 0.0 | 0.0 |
| Example 30 (1,6-HDO) | 2 | 12 | 0.005 | 90.1 | 83.7 | 6.4 | 0.0 | 0.0 | 0.0 | 0.0 |
| | 8 | 12 | 0.005 | 100.0 | 98.0 | 0.3 | 0.6 | 0.0 | 0.0 | 0.0 |
| Example 31 (1,2-PDO) | 2 | 12 | 0.005 | 98.6 | 76.3 | 18.9 | 2.7 | _0.7_ | 0.0 | 0.0 |
| | 8 | 12 | 0.005 | 99.3 | 81.6 | 13.8 | 3.2 | 0.7 | 0.0 | 0.0 |
| Example 32 (1,3-BDO) | 2 | 12 | 0.005 | 98.5 | 93.2 | 1.5 | 3.3 | 0.5 | 0.0 | 0.0 |
| | 8 | 12 | 0.005 | 100.0 | 96.0 | 0.0 | 3.5 | 0.5 | 0.0 | 00 |
| Example 33 (CHDM) | 2 | 12 | 0.005 | 32.5 | 0.0 | 32.5 | 0.0 | 0.0 | 0.0 | 0.0 |
| | 8 | 12 | 0.005 | 98.1 | 86.9 | 11.1 | 0.0 | 0.0 | 0.0 | 0.0 |
| Example 34 (2E1H) | 2 | 12 | 0.005 | 99.5 | 99.5 | 0.0 | 0.0 | 0.0 | 0.0 | 0.0 |
| | 8 | 12 | 0.005 | 100.0 | 100.0 | 0.0 | 0.0 | 0.0 | 0.0 | 0.0 |

DMT: Dimethyl terephthalate, $K_2$-LPA: Dipotassium terephthalate, K-MMT: Potassium monomethyl terephthalateEG: Ethylene glycol. PDO: Propanediol, BDO: Butanediol, HDO: Hexanediol,

CHDM: 1,4-Cyclohexanedimethanol, 2E1H: 2-Ethyl-1-hexanol Monomer product (monomer product): both methyt groups in DMT are completely exchanged by diol reactant Intermediate (intermediate): only one methyl group in DMT is exchanged by transesterification

EP 4 299 556 B1

As a result of transesterification using a chain diol (Example 12 and Examples 28 to 32) and a cyclic diol (Example 33) as additive reactants, the initial conversion rate of DMT was somewhat slowed as the number of carbon atoms was increased. This can be expected as a result that steric hindrance may occur as the molecular size of the diol increases, and the reaction activity for the transesterification reaction decreases as the number of carbon atoms increases. When 1,3-propanediol was used as a reactant of the transesterification reaction (Example 28), in the primary transesterification in which DMT was converted to 1-(3-hydroxypropyl) 4-methyl terephthalate (HBMT) which is a reaction intermediate, the reaction rate was observed to be faster than the case where 1,4-butanediol was used as a reactant of the transesterification reaction (Example 29), but in the secondary transesterification reaction of converting the reaction intermediate to the end product, bis-(3-hydroxypropyl) terephthalate (BHPT), the reaction rate was relatively slow. This shows that the structural characteristics of the diol compound substituted by the primary transesterification reaction affect the rate of the secondary transesterification reaction.

On the other hand, instead of 1,3-propanediol (Example 28) having diols ($\alpha,\omega$-diol) at respective ends of the chain thereof, when using as a reactant of the transesterification reaction, a structural isomer (1,2-propanediol) (Examples 31), which is expected to have an effect on a transesterification reaction because the second alcohol functional group is closely disposed, it was observed both the primary and secondary transesterification reaction rates were greatly limited. The results of the transesterification reactions using, as reactants, structural isomers of butanediol having a relatively long chain structure (Examples 29 and 32) were compared. It was observed that the rates of the primary and secondary transesterification reactions are not significantly limited due to the flexibility of the chain. When 1,4-cyclohexanedimethanol containing a relatively large cyclic hydrocarbon structure was applied as a reactant (Example 33), the transesterification reaction proceeded slowly due to the steric hindrance of the molecular structure of the diol itself. On the other hand, it was observed that only the primary transesterification reaction proceeds selectively at the beginning of the reaction. This result shows that when different types of monohydric or polyhydric alcohol reactants are used for the primary transesterification reaction and the secondary transesterification reaction, it is also possible to design monomer structures having different ester bond pairs.

When transesterification was carried out using 2-ethylhexanol, which is the main raw material of a plastic plasticizer, as an additive reactant (Example 34), the conversion of DMT proceeded rapidly from the beginning of the reaction, and most of DMT was converted within 2 hours. Thereafter, dioctyl terephthalate (DOTP), which is a monomer product, was stably maintained without generation of by-products even when exposed to high temperature reaction conditions for 8 hours.

In summary, according to an example of the present application, when transesterification is performed under conditions in which different types of alcohols are used, monomers in which functional groups of various structures are bonded to terephthalate by ester bonds can be produced.

Although specific features of the present disclosure have been described in detail above, it will be clear to those skilled in the art that these specific features are only preferred embodiments, and the scope of the present disclosure is not limited thereby. Accordingly, the substantial scope of the present disclosure will be defined by the appended claims.

## Claims

1. A method for producing a terephthalate derivative from dimethyl terephthalate, the method comprising:

   (a) carrying out a transesterification reaction by adding a monohydric and/or polyhydric alcohol to DMT as a reactant for transesterification, while applying a flow of carrier gas in the presence of one or more transesterification reaction catalysts selected from the group consisting of alkali carbonates, alkali hydroxides, alkali alkoxides, alkaline earth metal oxides, and guanidine-based organic; and
   (b) separating and obtaining the terephthalate derivative produced by the reaction.

2. The method of claim 1, wherein the transesterification reaction catalyst is used in an amount of 0.00005 to 1.0 mol per 1 mol of the dimethyl terephthalate.

3. The method of claim 1, wherein the alcohol in the step (a) is ethylene glycol.

4. A method for producing a terephthalate derivative from a polymer containing an ester functional group, the method comprising:

   (A) depolymerizing the polymer containing an ester functional group by adding an alcohol, a polar aprotic solvent, and potassium carbonate ($K_2CO_3$) to the polymer containing an ester functional group, wherein the alcohol is methanol, and wherein a depolymerization product is obtained including DMT;

(B) causing a transesterification reaction of the depolymerization product obtained in the step (A) by adding a monohydric and/or polyhydric alcohol to the depolymerization product including DMT as a reactant for transesterification while applying a flow of carrier gas in the presence of one or more transesterification reaction catalysts selected from the group consisting of alkali carbonates, alkali hydroxides, alkali alkoxides to the depolymerization product; and

(C) separating and obtaining the terephthalate derivative produced by the reaction.

5. The method of claim 4, wherein after performing the step (A), one or more compounds are separated from the depolymerization product and discharged to the outside.

6. The method of claim 5, wherein the one or more compounds discharged to the outside comprise unreacted polymer containing ester functional group, the undissolved catalyst, and polar aprotic solvent, and by-products of the reaction.

7. The method of claim 4, wherein the polar aprotic solvent in the step (A) is an inert solvent that does not participate in the depolymerization reaction of the polymer containing an ester functional group, serves to reduce the solubility of the catalyst in the alcohol, and is a compound in which at least one of halogen, oxygen, and nitrogen is bonded to an organic compound that has a skeletal structure having a chain form and/or a ring form.

8. The method of claim 4, wherein the polar aprotic solvent in the step (A) is at least one selected from the group consisting of toluene, xylene, acetone, methyl ethyl ketone, methyl isobutyl ketone, acetonitrile, propionitrile, aminopropionitrile, methylaminopropionitrile, iminodipropionitrile, butyronitrile, methylbutenenitrile, butanenitrile, methylethylether, diethylether, ethylphenylether, dimethoxybenzene, trimethoxybenzene, methoxyphenol, tetrahydrofuran, methyltetrahydrofuran, dioxane, chloromethane, dichloromethane, chloroform, tetrachloromethane, chlorobenzene, dichlorobenzene, and trichlorobenzene.

9. The method of claim 4, wherein in the step (A), compared to the number of moles of a repeating unit of the polymer containing an ester functional group, the number of moles of the alcohol and the number of moles of the polar aprotic solvent is respectively 0.1 to 5,000 times the number of moles of the repeating unit of the polymer containing an ester functional group.

10. The method of claim 4, wherein before performing the transesterification reaction of the step (B), each of the number of moles of the monohydric and/or polyhydric alcohol as a reactant for a transesterification reaction of the depolymerization product of the step (A) and the number of moles of at least one transesterification catalysts selected from the group consisting of alkali carbonates, alkali hydroxides, alkali alkoxides, alkaline earth metal oxides, and guanidine-based organic compounds is controlled to fall within a predetermined range in relative to the number of moles of dimethyl terephthalate contained in the depolymerization product.

11. The method of claim 4, wherein the methanol recovered in the step (C) is reused as a raw material for the depolymerization of the step (A).

12. The method of claim 4, wherein the number of moles of the transesterification reaction catalyst controlled in the step (B) is 0.00005 to 1.0 times the number of moles of the dimethyl terephthalate contained in the depolymerization product.

**Patentansprüche**

1. Verfahren zur Herstellung eines Terephthalatderivats aus Dimethylterephthalat, wobei das Verfahren umfasst:

(a) Durchführen einer Umesterungsreaktion durch Zugeben eines einwertigen und/oder mehrwertigen Alkohols zu DMT als Reaktionspartner für die Umesterung, während gleichzeitig ein Trägergasstrom in Gegenwart von einem oder mehreren Umesterungsreaktionskatalysatoren zum Einsatz kommt, die aus der Gruppe bestehend aus Alkalicarbonaten, Alkalihydroxiden, Alkalialkoxiden, Erdalkalimetalloxiden und einer organischen Verbindung auf Basis von Guanidin ausgewählt sind; und

(b) Abtrennen und Gewinnen des Terephthalatderivats, das mit der Reaktion hergestellt wurde.

2. Verfahren nach Anspruch 1, wobei der Umesterungsreaktionskatalysator in einer Menge von 0,00005 bis 1,0 Mol je 1 Mol Dimethylterephthalat verwendet wird.

3. Verfahren nach Anspruch 1, wobei der Alkohol in Schritt (a) Ethylenglycol ist.

4. Verfahren zur Herstellung eines Terephthalatderivats aus einem Polymer, das eine funktionelle Estergruppe enthält, wobei das Verfahren umfasst:

(A) Depolymerisieren des Polymers, das eine funktionelle Estergruppe enthält, durch Zugeben eines Alkohols, eines polaren aprotischen Lösungsmittels und von Kaliumcarbonat ($K_2CO_3$) zu dem Polymer, das eine funktionelle Estergruppe enthält, wobei der Alkohol Methanol ist und wobei ein Depolymerisationsprodukt gewonnen wird, das DMT aufweist;
(B) Bewirken einer Umesterungsreaktion des Depolymerisationsprodukts, das in Schritt (A) gewonnen wurde, durch Zugeben eines einwertigen und/oder mehrwertigen Alkohols zu dem Depolymerisationsprodukt, das DMT aufweist, als Reaktionspartner für die Umesterung, während gleichzeitig ein Trägergasstrom in Gegenwart von einem oder mehreren Umesterungsreaktionskatalysatoren, die aus der Gruppe bestehend aus Alkalicarbonaten, Alkalihydroxiden, Alkalialkoxiden ausgewählt sind, bei dem Depolymerisationsprodukt zum Einsatz kommt; und
(C) Abtrennen und Gewinnen des Terephthalatderivats, das mit der Reaktion hergestellt wurde.

5. Verfahren nach Anspruch 4, wobei nach dem Durchführen von Schritt (A) ein oder mehrere Verbindungen von dem Depolymerisationsprodukt abgetrennt und nach außen abgeführt werden.

6. Verfahren nach Anspruch 5, wobei die eine oder die mehreren Verbindungen, die nach außen abgeführt werden, nicht zur Reaktion gebrachtes Polymer, das eine funktionelle Estergruppe enthält, den nicht gelösten Katalysator und polares aprotisches Lösungsmittel sowie Nebenprodukte der Reaktion umfassen.

7. Verfahren nach Anspruch 4, wobei das polare aprotische Lösungsmittel in Schritt (A) ein inertes Lösungsmittel ist, das nicht an der Depolymerisationsreaktion des Polymers beteiligt ist, das eine funktionelle Estergruppe enthält, zum Verringern der Löslichkeit des Katalysators im Alkohol dient und eine Verbindung ist, in der eines von Halogen, Sauerstoff und Stickstoff an eine organische Verbindung gebunden ist, die eine Gerüststruktur mit einer Kettenform und/oder einer Ringform aufweist.

8. Verfahren nach Anspruch 4, wobei das polare aprotische Lösungsmittel in Schritt (A) mindestens eins ist, das aus der Gruppe bestehend aus Toluol, Xylen, Aceton, Methylethylketon, Methylisobutylketon, Acetonitril, Propionitril, Aminopropionitril, Methylaminopropionitril, Iminodipropionitril, Butyronitril, Methylbutenennitril, Butanennitril, Methylethylether, Diethylether, Ethylphenylether, Dimethoxybenzol, Trimethoxybenzol, Methoxyphenol, Tetrahydrofuran, Methyltetrahydrofuran, Dioxan, Chlormethan, Dichlormethan, Chloroform, Tetrachlormethan, Chlorbenzol, Dichlorbenzol und Trichlorbenzol ausgewählt ist.

9. Verfahren nach Anspruch 4, wobei in Schritt (A) die Molanzahl des Alkohols beziehungsweise die Molanzahl des polaren aprotischen Lösungsmittels im Vergleich zur Molanzahl einer Wiederholungseinheit des Polymers, das eine funktionelle Estergruppe enthält, das 0,1- bis 5.000-Fache der Molanzahl der Wiederholungseinheit des Polymers entspricht, das eine funktionelle Estergruppe enthält.

10. Verfahren nach Anspruch 4, wobei vor dem Durchführen der Umesterungsreaktion von Schritt (B) sowohl die Molanzahl des einwertigen und/oder mehrwertigen Alkohols als Reaktionspartner für eine Umesterungsreaktion des Depolymerisationsprodukts von Schritt (A) als auch die Molanzahl von mindestens einem Umesterungskatalysator, der aus der Gruppe bestehend aus Alkalicarbonaten, Alkalihydroxiden, Alkalialkoxiden, Erdalkalimetalloxiden und organischen Verbindungen auf Basis von Guanidin ausgewählt ist, so gesteuert wird, dass sie bezogen auf die Molanzahl von Dimethylterephthalat, das im Depolymerisationsprodukt enthalten ist, in einen vorgegebenen Bereich fällt.

11. Verfahren nach Anspruch 4, wobei das Methanol, das in Schritt (C) erhalten wird, als Ausgangsmaterial für den Depolymerisationsschritt von Schritt (A) wiederverwendet wird.

12. Verfahren nach Anspruch 4, wobei die Molanzahl des Umesterungsreaktionskatalysators, die in Schritt (B) gesteuert wird, das 0,00005- bis 1,0-Fache der Molanzahl des Dimethylterephthalats entspricht, das im Depolymerisationsprodukt enthalten ist.

**Revendications**

1. Procédé de production d'un dérivé de téréphtalate à partir de diméthyl téréphtalate, le procédé comprenant :

   (a) la réalisation d'une réaction de transestérification en ajoutant un alcool monohydrique et/ou polyhydrique au DMT en tant que réactif de transestérification, tout en appliquant un flux de gaz porteur en présence d'un ou de plusieurs catalyseurs de réaction de transestérification choisis dans le groupe constitué de carbonates alcalins, d'hydroxydes alcalins, d'alcoxydes alcalins, d'oxydes de métaux alcalino-terreux et d'un composé organique à base de guanidine ; et
   (b) la séparation et l'obtention du dérivé de téréphtalate produit par la réaction.

2. Procédé selon la revendication 1, dans lequel le catalyseur de réaction de transestérification est utilisé dans une quantité de 0,00005 à 1,0 mole pour 1 mole de diméthyl téréphtalate.

3. Procédé selon la revendication 1, dans lequel l'alcool dans l'étape (a) est de l'éthylène glycol.

4. Procédé de production d'un dérivé de téréphtalate à partir d'un polymère contenant un groupement fonctionnel ester, le procédé comprenant :

   (A) la dépolymérisation du polymère contenant un groupement fonctionnel ester en ajoutant un alcool, un solvant aprotique polaire et du carbonate de potassium ($K_2CO_3$) au polymère contenant un groupement fonctionnel ester, dans lequel l'alcool est du méthanol, et dans lequel un produit de dépolymérisation est obtenu comportant du DMT ;
   (B) la réalisation d'une réaction de transestérification du produit de dépolymérisation obtenu dans l'étape (A) en ajoutant un alcool monohydrique et/ou polyhydrique au produit de dépolymérisation comportant le DMT en tant que réactif de transestérification en appliquant un flux de gaz porteur en présence d'un ou de plusieurs catalyseurs de réaction de transestérification choisis dans le groupe constitué de carbonates alcalins, d'hydroxydes alcalins, d'alcoxydes alcalins au produit de dépolymérisation ; et
   (C) la séparation et l'obtention du dérivé de téréphtalate produit par la réaction.

5. Procédé selon la revendication 4, dans lequel, après la réalisation de l'étape (A), un ou plusieurs composés sont séparés du produit de dépolymérisation et déchargés vers l'extérieur.

6. Procédé selon la revendication 5, dans lequel le ou les composés déchargés vers l'extérieur comprennent du polymère contenant un groupement fonctionnel ester n'ayant pas réagi, le catalyseur non dissout et du solvant aprotique polaire, et des produits secondaires de la réaction.

7. Procédé selon la revendication 4, dans lequel le solvant aprotique polaire dans l'étape (A) est un solvant inerte qui ne participe pas à la réaction de dépolymérisation du polymère contenant un groupement fonctionnel ester, sert à réduire la solubilité du catalyseur dans l'alcool, et est un composé dans lequel au moins un parmi un halogène, l'oxygène et l'azote est lié à un composé organique qui a une structure squelettique ayant une forme en chaine et/ou une forme d'anneau.

8. Procédé selon la revendication 4, dans lequel le solvant aprotique polaire dans l'étape (A) est au moins un choisi dans le groupe constitué du toluène, du xylène, de l'acétone, de la méthyl éthyl cétone, de la méthyl isobutyl cétone, de l'acétonitrile, du propionitrile, de l'amino propionitrile, du méthylamino propionitrile, de l'iminodipropio nitrile, du butyronitrile, du méthyl butène nitrile, du butanène nitrile, du méthyléthyl éther, du diéthyléther, de l'éthylphényl éther, du diméthoxy benzène, du triméthoxy benzène, du méthoxyphénol, du tétrahydrofurane, du méthyl tétrahydrofuran, du dioxane, du chlorométhane, du dichlorométhane, du chloroforme, du tétrachlorométhane, du chlorobenzène, du dichlorobenzène, et du trichlorobenzène.

9. Procédé selon la revendication 4, dans lequel, dans l'étape (A), comparativement au nombre de moles d'unités de répétition du polymère contenant un groupement fonctionnel ester, le nombre de moles d'alcool et le nombre de moles de solvant aprotique polaire sont respectivement égaux à 0,1 à 5.000 fois le nombre de moles de l'unité de répétition du polymère contenant un groupement fonctionnel ester.

10. Procédé selon la revendication 4, dans lequel, avant d'effectuer la réaction de transestérification de l'étape (B) , chacun des nombres de moles d'alcool monohydrique et/ou polyhydrique servant de réactif pour une réaction de transestérification du produit de dépolymérisation dans l'étape (A) et le nombre de moles d'au moins un des catalyseurs de transestérification choisi dans le groupe constitué de carbonates alcalins, d'hydroxydes alcalins, d'alcoxydes alcalins, d'oxydes de métaux alcalino-terreux et de composés organiques à base de guanidine est réglé pour tomber dans une plage prédéterminée en relation avec le nombre de moles de diméthyl téréphtalate contenues dans le produit de dépolymérisation.

11. Procédé selon la revendication 4, dans lequel le méthanol récupéré dans l'étape (C) est réutilisé en tant que matière première pour la dépolymérisation de l'étape (A).

12. Procédé selon la revendication 4, dans lequel le nombre de moles de catalyseur de réaction transestérification réglé dans l'étape (B) équivaut à 0,00005 à 1,0 fois au nombre de moles de diméthyl téréphtalate contenues dans le produit de dépolymérisation.

Fig. 1

Fig. 2

## (a) Closed-loop for carrier gas

## (b) Open-loop for carrier gas

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10287741 A

**Non-patent literature cited in the description**

- **AMARSINH L. JADHAV** ; **RADHIKA S. MALKAR** ; **GANAPATI D. YA**. Zn- and Ti-Modified Hydrotalcites for Transesterification of Dimethyl Terephthalate with Ethylene Glycol: Effect of the Metal Oxide and Catalyst Synthesis Method. *ACS Omega*, 2020, vol. 5, 2088-2096

- **D. DINH PHAM** ; **J. CHO**. Low-energy catalytic methanolysis of poly(ethyleneterephtalate). *Green Chemistry*, 2021, vol. 23, 511-525